# EUROPÄISCHE PATENTANMELDUNG

(11) **EP 1 398 388 A2**
(43) Veröffentlichungstag der Anmeldung: **17.03.2004**
(21) Anmeldenummer: 03017541.8
(22) Anmeldetag: 06.08.2003
(51) Int. Cl.: C12Q 1/68

(54) **Methode zur Bestimmung eines erblichen Thromboserisikos mit DNA-arrays**

(30) Priorität: 09.08.2002 DE 10237073
(71) Anmelder: OGHAM GmbH, 48149 Münster (DE)
(72) Erfinder: Cullen, Paul, 48149 Münster (DE); Seedorf, Udo, 48149 Münster (DE)
(74) Vertreter: König, Gregor Sebastian, Dipl.-Biol.

(57) **Zusammenfassung**

Verfahren zum Bestimmen der genetischen Disposition zur Thromboseneigung eines Patienten durch die Untersuchung mindestens zweier Nukleinsäureabschnitte aus dem Genom des Patienten und deren ausschließliche multifaktorielle Auswertung, wobei die Nukleinsäureabschnitte allelische Polymorphismen umfassen können, deren Vorhandensein im menschlichen Genom mit einer erhöhten Thromboseneigung korreliert.

## Beschreibung

Die vorliegende Erfindung betrifft eine Methode zur Untersuchung von Nukleinsäure-Polymorphismen, die mit dem erblichen Thromboserisiko korrelieren und nimmt die Priorität der deutschen Patentanmeldung 102 37 073.7 in Anspruch, auf die inhaltlich Bezug genommen wird.

Tiefe Beinvenenthrombosen sind häufig: sie treten bei ungefähr zwei von tausend Personen irgendwann im Laufe ihres Lebens auf. Solche tiefen Venenthrombosen sind oft mit ernsten Komplikationen verbunden. Zum einen wird durch eine Venenthrombose der Blutfluß beeinträchtigt, was zu einer schmerzhaften Schwellung führt. Außerdem kommt es zu einer lokalen Endzündungsreaktion. Zum anderen können sich Gerinnselfragmente oder der ganze Thrombus am Entstehungsort ablösen und mit dem venösen Blutstrom in die rechte Herzkammer und schließlich in die Pulmonalarterie gelangen. Diese Komplikation, eine sogenannte Lungenembolie, geht häufig mit einer drastischen Abnahme der Lungendurchblutung sowie des Gasaustausches einher und führt oft zum plötzlichen Tod des Betroffenen. Jedes Jahr versterben 40 000 Personen in Deutschland an den Folgen einer Lungenembolie.

In der Regel entsteht eine Thrombose entweder durch die pathologische Bildung eines Thrombus in Folge eines Defekts der Blutgerinnungskaskade oder durch eine Störung der Thrombolyse.

Das Gerinnungssystem im Plasma setzt sich aus verschiedenen enzymatischen Reaktionen zusammen, die schließlich zur Umwandlung von löslichem Fibrinogen zu unlöslichem Fibrin führen. Durch die Vernetzung des Fibrins entsteht der eigentliche Thrombus. Ein funktionierendes Blutgerinnungssystem ist lebenswichtig. Ohne Blutgerinnung würden selbst kleinste Verletzungen zu schwersten Blutungen führen. Die beteiligten Reaktionen müssen jedoch einer strengen räumlichen und zeitlichen Kontrolle unterliegen. Ein Blutgerinnsel darf nur dann entstehen, wenn eine Verletzung des Organismus vorliegt und muß außerdem auf die Verletzungsstelle beschränkt bleiben, um nicht den gesamten Blutkreislauf zu beeinträchtigen.

Die Gerinnungskaskade wird durch die Bindung von aktiviertem Faktor VII (FVIIa) an Gewebsfaktor (TF) ausgelöst. Gewebsfaktor ist ein membranständiges Protein, das erst durch die Verletzung des Endothels zugänglich wird. Der TF-Vlla-Komplex katalysiert eine limitierte Proteolyse der Proenzyme Faktor IX und Faktor X und wandelt diese in ihre aktiven Formen um (1). Aktivierter Faktor IX (FIXa) bindet an seinen Kofaktor VIIIa, dessen aktivierte Form aus dem Proenzym Faktor VIII entsteht. Der IXa-Vllla-Komplex aktiviert Faktor X sehr viel schneller als der TF-Vlla-Komplex. Somit kommt es zu einer verstärkten Bildung von aktiviertem Faktor X (FXa), der wiederum an seinen Kofaktor, aktivierten Faktor V (FVa), bindet, um so den Prothrombinase-Komplex zu bilden. Durch diesen Komplex wird Prothrombin (Faktor II, FII) proteolytisch in Thrombin umwandelt. Thrombin wandelt lösliches Fibrinogen in unlösliches Fibrin um, wodurch ein Blutgerinnsel entsteht (2,3). Neben der Umwandlung von Fibrinogen zu Fibrin, spaltet Thrombin Faktor V zu FVa und Faktor VIII zu FVllla. Außerdem wird auch Faktor IX möglicherweise von Thrombin aktiviert (4).

Die strenge zeitliche und räumliche Begrenzung der Gerinnungskaskade wird durch eine zweite Kaskade gerinnungshemmender und thrombolytischer Faktoren, die parallel zur Gerinnungskaskade aktiviert werden, sichergestellt. Allgemein handelt es sich bei diesen anti-thrombotischen Faktoren um Proteasen, die durch Spaltung der Gerinnungsfaktoren diese inaktivieren.

Einer der wichtigsten anti-thrombotischen Faktoren ist Protein C. Nach seiner Aktivierung ist Protein C, zusammen mit seinem Kofaktor Protein S, für die Proteolyse der Gerinnungsfaktoren FV, FVa, FVIII und FVllla verantwortlich (5-13).

Als Risiken für die Entstehung einer tiefen Venenthrombose gelten frische chirurgische Eingriffe, Krebs, Schwangerschaft, Übergewicht, langandauernde Immobilisierung der unteren Gliedmaßen, Rauchen und die Einnahme oraler Kontrazeptiva. In den letzten Jahren sind außerdem eine Reihe genetischer Merkmale identifiziert worden, die mit einer Zunahme des Thromboserisikos assoziiert sind:

### Factor V^{Leiden}

Neben seltenen erblichen Defekten von Protein C und Protein S, die bereits *in utero* zu schweren Komplikationen in Folge von Thrombosen führen, stellt eine einzige Mutation im Faktor V-Gen eine der wichtigsten Ursachen für ein erhöhtes Thromboserisiko in der Gesamtbevölkerung dar. Bei dieser Mutation handelt es sich um einen Austausch von Guanosin durch Adenosin an Nukleotid 1691 des Genes (G1691A), der zu einer Substitution von Arginin durch Glutamin im Kodon 506 des Faktor V-Proteins führt (14-18).

Diese Arg⁵⁰⁶→Gln (R506Q)-Variante, die nach der niederländischen Stadt, in der sie entdeckt wurde, Faktor V^{Leiden} genannt wird, kommt überraschend häufig vor und stellt die häufigste Ursache für eine tiefe Venenthrombose dar. In den meisten Bevölkerungen beträgt die Allelfrequenz mindestens 0,5%, in Deutschland liegt sie bei ungefähr 1,5%. Dies bedeutet, daß ungefähr 3% der deutschen Bevölkerung heterozygote Träger der Faktor V^{Leiden} Mutation sind. Träger das Faktor-V-Protein die Leiden-Mutation, so wird es gegenüber der proteolytischen Aktivität von aktiviertem Protein C resistent (sogenannte APC-Resistenz). Dies wiederum führt zu einer gesteigerten Aktivierung der Gerinnungskaskade und somit zu einer erhöhten Thromboseneigung. Mehr als 95% aller Fälle von APC-Resistenz sind auf die FV^{Leiden}-Mutation zurückzuführen.

Neben dem genetischen Nachweis der FV^{Leiden} Mutation, kann die APC-Resistenz auch mittels biochemischer Methoden festgestellt werden. Der genetische Test hat jedoch den Vorteil, daß er zwischen homozygoten und heterozygoten Trägern unterscheiden kann. Diese Unterschied ist wichtig: während Heterozygote ohne andere Risikofaktoren im Vergleich zur Normalbevölkerung ein 5-10-fach erhöhtes Thromboserisiko aufweisen, steigt das Risiko bei Homozygoten um das 50-100-fache an. Außerdem wird der genetische Test durch Faktoren, die die biochemische Untersuchung stören können, nicht beeinflusst. Zu solchen Faktoren zählen eine Behandlung mit gerinnungshemmenden Mitteln, die Einnahme von oralen Empfängnisverhütungsmittel oder die Hormonersatztherapie. Falls weitere Risikofaktoren wie oben erwähnt vorliegen, kommt es sowohl bei heterozygoten als auch bei homozygoten Trägern der Faktor V^{Leiden}-Mutation zu einer zusätzlichen Steigerung des Thromboembolie-Risikos (19).

### Faktor V^{Cambridge}

Kürzlich wurden weitere Mutationen des Faktor V-Proteins, die mit einer Venenthrombose assoziiert sind, beschrieben.

Eine dieser Mutationen wurde zum ersten Mal im Addenbrooke's Hospital an der Universität von Cambridge in England beschrieben und wird daher Faktor V^{Cambridge} genannt. Bei der Faktor V^{Cambridge}-Mutation handelt es sich um eine Transversion von Guanosin zu Cytosin an der zweiten Base von Kodon 306 (G1091C), die zu einer Substitution von Threonin durch Arginin an dieser Position führt (R306T). Auch diese Mutation verändert eine Stelle im Faktor V-Protein, die durch aktiviertes Protein C gespalten wird. Obwohl die genaue Prävalenz von Faktor V^{Cambridge} unbekannt ist, ist diese Mutation wahrscheinlich sehr selten. So wurde beispielsweise in der ersten Studie, die diese Veränderung beschrieb, die Mutation in einer Gruppe von 226 Blutspendern nicht gefunden (20). Faktor V^{Cambridge} scheint einen ähnlichen Phänotyp wie Faktor V^{Leiden} aufzuweisen. Es ist sehr wahrscheinlich, daß die Faktor V^{Cambridge}-Mutation mit anderen Risikofaktoren in gleicher Weise wie Faktor V^{Leiden} interagiert.

### G20210A Mutation im Prothrombin (Faktor II)-Gen

Nach Faktor V^{Leiden} stellt eine G zu A Transition an Nukleotidposition 20210 im nicht-translatierten 3'-Bereich des Prothrombin (Faktor ll)-Gens die wichtigste Mutation dar, die eine erbliche Neigung zur Thrombophilie und tiefen Venenthrombose verursacht (21). Vermutlich führt diese Mutation im Gen-Promoter dazu, daß die Konzentration des Prothrombins im Blut um circa 15% ansteigt. Zwischen 1% und 3% der europäischen Bevölkerung sind Träger der Faktor II^{G20210A}-Mutation. Es wird angenommen, daß solche Träger ein etwa dreifach erhöhtes Risiko für tiefe Venenthrombose aufweisen. Wie bei anderen gerinnungsfördernden Mutationen führt die Kombination von Faktor II^{G20210A} mit anderen Risikofaktoren zu einer erheblichen Zunahme des Thromboserisikos. Beispielsweise wird angenommen, daß das Risiko für Frauen mit Faktor II^{G20210A}, die orale Kontrazeptiva nehmen, im Vergleich zur normalen Bevölkerung um mehr als das 100-fache erhöht ist (22, 23).

Ungefähr 30% der Patienten mit tiefen Venenthrombosen, die Träger von Faktor V^{Leiden} sind, sind außerdem heterozygot für die Mutation G20210A in der 3'-untranslatierten Region des Prothrombin-Gens. Eine kombinierte Trägerschaft für Faktor V^{Leiden} und Faktor II^{G20210A} ist wahrscheinlich mit einer sehr großen Zunahme des Thromboserisikos assoziiert (22,24-26). Im Gegensatz zu Faktor V^{Leiden} scheint Faktor II^{G20210A} außerdem mit einem ungefähr 4-6-fach erhöhten Risiko für Herzinfarkt assoziiert zu sein (27,28). Ein weiterer wichtiger Unterschied zwischen Faktor II^{G20210A} und Faktor V^{Leiden} ist, daß es keinen biochemischen Test für die Prothrombin-Mutation gibt, weswegen der Nachweis nur auf genetischem Wege möglich ist (29).

### 4G/5G-Polymorphismus im Promoter des Plasminogen-Aktivator-Inhibitor-Gens

Plasmin ist ein Enzym, das Fibrin spaltet und auf diese Weise Blutgerinnsel auflöst. Plasmin wird durch Aktivierung des Proenzyms Plasminogen gebildet. Diese Aktivierung kann mittels des Enzyms Urokinase (u-PA), das überwiegend in der Niere zu finden ist, erfolgen. Häufiger jedoch ist die Aktivierung durch Gewebsplasminogenaktivator (t-PA), der in großen Mengen in den Endothelzellen der Blutgefäßwand vorliegt. t-PA zeigt eine hohe Bindungsaffinität zum Fibrin. Nach dieser Bindung kommt es zu einer deutlichen Steigerung der t-PA-Aktivität (30). Die Plasminogenaktivatoren werden wiederum durch Plasminogen-Aktivator-inhibitor I (PAI-1), einem Serinproteaseinhibitor (Serpin), der hauptsächlich in der Leber exprimiert wird, inhibiert (31,32). Im Jahre 1993 wurde im Promoter des PAI-1-Gens ein häufiger Polymorphismus entdeckt (33). Dieser Polymorphismus, die Insertion/Deletion einer einzelnen Base 675 Basenpaare stromaufwärts vom Transkriptionsstart, führt zum Auftreten von 4 oder 5 Gunanosinresten in diesem Bereich. Das 4G-Allel hat eine Häufigkeit von circa 40%, das 5G-AI-lel von circa 60% in der Gesamtbevölkerung (33). Träger des 4G-Allels weisen einen höheren PAI-1-Spiegel auf. Die vermutliche Ursache hierfür ist, daß ein Transkriptionsrepressor, der an das 5G-Allel bindet, nicht an das 4G-Allel binden kann, was zu einer erhöhten Promoteraktivität bei 4G-Trägern führt (33,34).

Das Auftreten des 4G-Allels von PAI-1 ist mit einer leichten Zunahme des Herzinfarktrisikos assoziiert (35). Für lange Zeit herrschte jedoch Uneinigkeit darüber, ob das 4G-Allel mit einem erhöhten Risiko für venöse Thromboembolien in Zusammenhang steht: einige Studien zeigten ein erhöhtes Thromboserisiko (36), andere nicht (37-39). Neuere Studien haben Licht in diese Kontroverse gebracht. Bei Patienten mit tiefer Beinvenenthrombose fanden Seguí und Kollegen keine Anhäufung des 4G-Allels im Vergleich zu normalen Kontrollen. Bei Patienten mit anderen vererbten thrombophilischen Defekten, insbesondere Faktor V^{Leiden} und Prothrombin^{G20210A}, führte jedoch das zusätzliche Auftreten des 4G-Allels zu einer Steigerung des Thromboserisikos: so war die Kombination von 4G mit anderem genetischen Defekt bei Patienten mit tiefer Beinvenenthrombose achtmal häufiger als in normalen Kontrollen (40). Das 4G-Allel ist also eindeutig ein Faktor, der eine bereits vorliegende Prädisposition zur Thrombose deutlich verschärft. Das Vorliegen anderer nicht genetischer Thromboserisikofaktoren, wie Schwangerschaft oder eine kürzliche Operation, vergrößert den Effekt des 4G-Phänotyps von PAI-1 dagegen nicht (40).

### Homocystein-Stoffwechsel und venöse Thrombose

Die klassische Homocystinurie in eine seltene erbliche Stoffwechselkrankheit, die auf Defekte in den Enzymen Cystathionin-beta-Synthase (CBS) oder Methylentetrahydrofolat-Reduktase (MTHFR) zurückzuführen ist. Das Krankheitsbild wird durch sehr hohe Serum- und Harnspiegel von Methionin und Homocystein charakterisiert. Homocystein ist eine Aminosäure, die nicht in Proteinen vorkommt, aber Bestandteil des Methionin-Stoffwechsels ist. Die wichtigsten klinischen Zeichen der Homocystinurie betreffen die Augen, die Blutgefäße, das Skelett sowie das zentrale Nervensystem. Die Homocysteinurie ist mit einem erhöhten Risiko für Arteriosklerose und Venenthrombose assoziiert. In den letzten zehn Jahren hat sich jedoch herausgestellt, daß bereits eine geringfügige Erhöhung des Serum-Homocysteinspiegels mit einem erhöhten Risiko für eine Venenthrombose einhergeht (41,42). Die Ursache hierfür ist nicht bekannt. Geringfügig erhöhte Homocysteinspiegel können durch Gabe von Vitamin B6 (Pyridoxin) und Folsäure behandelt werden. In einigen Fällen kann auch die Gabe von Vitamin B12 (Hydrxycobalamin) notwendig sein (43). MTHFR katalysiert die Umwandlung von 5,19-Methylentetrahydrofolat zu 5-Methyltetrahydrofolat, dem Methylgruppendonor für die B12-abhängige Remethylierung von Homocystein zu Methionin. Vitamin B6 ist Kofaktor letzterer Reaktion.

### C677T und A1298C Polymorphismen im MTHFR-Gen

Im MTHFR-Gen sind zwei Polymorphismen, die zu einer milden Hyperhomocysteinämie beitragen können, beschrieben worden. Die C677T-Transition (Alanin-zu-Valin-Austausch an Kodon 222) führt zu einer erhöhten Thermolabilität des Enzyms und zu geringfügiger Reduzierung seiner *in vitro* Aktivität. Dieser Polymorphismus wurde zuerst von Kang et al. (44) beschrieben und weist eine Allelfrequenz von circa 0,4 auf (45). Personen, die homozygot für die C677T-Mutation sind, weisen leicht erhöhte Homocysteinspiegel auf, besonders dann, wenn ihr Folatspiegel niedrig ist (46). Eine Homozygotie für den C677T-Polymorphismus wurde in manchen (47,48), aber nicht allen Studien (45,49,50) als Risikofaktor für Venenthrombose beschrieben. Kürzlich ist ein weiterer Polymorphismus im MTHFR-Gen entdeckt worden, der mit einer verringerten *in vitro* Aktivität des Enzyms einhergeht (51,52). Diese Mutation, eine A-zu-C-Transversion des Nukleotids 1298, führt zu einem Austausch von Alanin durch Glutamin an Kodon 429 und weist eine Allelfrequenz von 0,33 auf. Nach Abwägung der bis jetzt vorliegenden Ergebnisse scheint weder eine Homo- noch eine Heterozygotie für die A1298C-Mutation allein mit einer Erhöhung des Homocysteinspiegels assoziiert zu sein. Eine gemischte Heterozygotie für C677T und A1298C jedoch scheint mit einer Verminderung der MTHFR-Aktivität und mit höheren Homocysteinspiegeln einherzugehen als eine alleinige Heterozygotie für eine der beiden Varianten (51). Da beide mutierten Allele in der Bevölkerung häufig vorkommen, ist eine solche gemischte Heterozygotie keinesfalls selten.

### 68 Basenpaar-Insertion an Nukleotid 844 des CBS-Gens

Mutationen im CBS-Gen verursachen klassischerweise eine Homocystinurie. Sebastio und Kollegen identifizierten jedoch 1995 in Neapel eine 68 bp-Insertion im Exon 8 des CBS-Gens bei italienischen Patienten mit einer Hyperhomocystinurie (53). Diese Mutation, die bei circa 8% der europäischen Bevölkerung vorkommt, hat allein nur einen geringfügigen Effekt auf den Homocystinspiegel. Allerdings kompensiert das Auftreten der 68 bp-Insertion im CBS-Gen die Homocystin-steigernde Wirkung in Personen, die gleichzeitig Träger des C677T-Polymorphismus im MTHFR-Gen sind.

### A2756G-Polymorphismus im Methionin-Synthase-Gen

Leclerc und Kollegen klonierten im Jahre 1993 das Gen der humanen Methionin-Synthase, auch Methyltetrahydrofolat-L-Homocystein-S-Methyltransferase (MTR) genannt (54) und identifizierten gleichzeitig einen Polymorphismus, A2756G, in diesem Gen. Der Polymorphismus führt zu einer Substitution von Glycin durch Aspartat an Kodon 919 und tritt mit einer Allelfrequenz von circa 16% auf. Diese Variante, die mit pathologischen Veränderungen der Koronararterien bei Rauchern in Zusammenhang gebracht wurde (55) und zu einer Abnahme des Kolonkarzinomrisikos führt (56), ist mit einer Zunahme des Homocystinspiegels assoziiert (57). Außerdem scheint der C677T-Polymorphismus im MTHFR-Gen und der A2756G-Polymorphismus im MTR-Gen einen additiven Effekt auf den Homocystinspiegel im Serum zu haben (57).

Es ist bekannte, insbesondere Risikogruppen - wie Frauen, die orale Kontrazeptiva nehmen, Rauchern, immobilisierten Personen oder Patienten, die sich einer Operation unterziehen - auf das Vorhandensein von genetischen Defekten, die mit Thrombose assoziiert sind, zu untersuchen.

In dem Patent EP 0 696 325 B1 ist beispielsweise ein Test zur Untersuchung des Faktors V^{Leiden} beschrieben, der mit einer Thrombose und / oder einer geringen antikoagulierenden Reaktion auf aktiviertes Protein C assoziiert ist (58).

Augenblicklich erfordert die Suche nach solchen Veränderungen die Amplifikation des polymorphen Abschnitts der genomischen DNA, üblicherweise unter Anwendung der Polymerasekettenreaktion (PCR). Dieser Amplifikation folgt die Detektion des Polymorphismus. Diese basiert auf einer Fragmentanalyse mit Hilfe der Gelelektrophorese und schließt gegebenenfalls eine vorgeschaltete Spaltung mit Restriktionsenzymen ein (für eine allgemeine Beschreibung dieser Technik vgl. (59)). Alternativ kann der Polymorphismus mit Hilfe einer spezifischen Hybridisierung markierter Sonden mit der amplifizierten DNA identifiziert werden. Jedes DNA-Fragment kann durch eine separate PCR-Reaktion isoliert werden oder alle Fragmente werden gemeinsam in einer einzigen Multiplex-PCR-Reaktion amplifiziert; wie es Ulvik und Ueland für die simultane Amplifikation von Einzelnukleotid-Polymorphismen beschrieben haben, die den Homocystein-Stoffwechsel betreffen (60). Andere Methoden zur Amplifikation sind die Ligandenkettenreaktion. Die Detektion kann außerdem mit Hilfe verzweigtkettiger DANN (branched DNA), mit Mikropartikel oder basierend auf der Fähigkeit des amplifizierten Produkts zur Ausbildung stabiler verzweigter Strukturen erfolgen, wie in der Patentanmeldung WO 99/36574 beschrieben (61).

Wie bereits ausgeführt, sind die üblichen Methoden zur Untersuchung einer genetischen Veranlagung für ein erhöhtes Thromboserisiko derzeit darauf beschränkt, den positiven oder negativen Nachweis jedes einzelnen allelen Polymorphismus zu führen. Da das genetische Gesamtrisiko allerdings von der Koexistenz verschiedener alleler Polymorphismen abhängt, stellen diese Methoden kein geeignetes Werkzeug dar, um das genetische Risiko einer Thrombose in seiner Vollständigkeit aussagekräftig beurteilen zu können. Beispiele für diese synergische Wirkung verschiedener, die Ausbildung einer Thrombose begünstigender genetischer Faktoren sind das 4G-Allel des PAI-1-Gens (das Auftreten des 4G-Allels zusammen mit Faktor V^{Leiden} oder Prothrombin^{G20210A} war in Patienten mit tiefer Venenthrombose achtmal höher als in Kontrollpersonen) oder die Interaktion von Faktor V^{Leiden} mit prothrombin^{G20210A} (siehe oben).

Aufgabe der vorliegenden Erfindung ist es daher, ein Mittel und eine Methode zur Bestimmung der genetische Veranlagung zu einem erhöhten Thromboserisiko bereitzustellen, wobei die synergistischen Effekte möglicher koexistierender alleler Varianten erfaßt werden können, die aber jeweils auch für sich allein zu einem erhöhten Thromboserisiko beitragen. Eine weitere Aufgabe der vorliegenden Erfindung ist es, ein Set molekulabiologischer Materialien bereitszustellen, die die Durchführung der in der Erfindung beschriebenen Methode ermöglichen.

Im Kern basiert sie vorliegende Erfindung darauf, das Thromboserisiko mit Hilfe eines Verfahrens zu bestimmen, das die Bestimmung mindestens zweier alleler Varianten ermöglicht, die relevant für die genetische Thrombose-Prädisposition sind. Die Untersuchungsergebnisse werden ausschließlich multifaktoriell ausgewertet.

Das erfindungsgemäße Verfahren - d.h. die simultane Untersuchung mindestens zweier wichtiger Polymorphismen, die die Entstehung einer Thrombophilie beeinflussen, in nur einem Testkit - macht die Anwendung einer Vielzahl der bisher bekannten Testsysteme überflüssig, die jedes nur ein einziges dieser Allele erfassen konnten. Damit liefert die, erfindungsgemäße Methode nicht nur ein sinnvolles System zur Risikoabschätzung, sondern ermöglicht außerdem eine Kosten- und Zeitersparnis.

Den spezifische Vorteil der erfindungsgemäßen Methode veranschaulicht folgendes Beispiel: Faktor V^{Leiden} hat eine Allelfrequenz von 1,5%. Die Allelfrequenz der Faktor II^{G20210A}-Mutation liegt zwischen 1 und 3%. Unter der begründeten Annahme, dass zwischen diesen Allelen keine Kopplung vorliegt, ist anzunehmen, dass 1 bis 3 von 2000 Personen in der europäischen Bevölkerung Träger beider Allele sind. Das Auftreten von Kombinationen aus den Polymorphismen, die die Gene des Homocysteinstoffwechsels betreffen, ist sehr viel wahrscheinlicher, berücksichtigt man die oben beschriebenen relativ hohen Allelfrequenz dieser Polymorphismen in der europäischen Bevölkerung. Beispielsweise hat der C677T-Polymorphismus im MTHFR-Gen eine Allelfrequenz von 0,40 der A2756G-Polymorphismus im MTR-Gen weist einen Wert von 0,16 auf. Dies lässt unter der Annahme, das keinerlei Kopplung beider Gene vorliegt, erwarten, daß beide Allele mit einer Wahrscheinlichkeit von 16% zusammen auftreten. Dehnt man diese Betrachtung auf mehrere Faktoren aus, ist bei einer dreifachen Kombination aus C677T-Polymorphismus bei MTHFR, A2756G bei MTR und Faktor V^{Leiden} mit einer Auftretenswahrscheinlichkeit von 5 unter 1000 Personen in der Gesamtbevölkerung zu rechnen. Mit Hilfe der vorliegenden Erfindung ist eine einfache Untersuchung dieser Patienten möglich.

Das parallele und gleichzeitige Screenen wichtiger Thrombophilie-relevanter Polymorphismen auf nur einem Array bietet die Möglichkeit, klinisch bedeutsame und bis heute unbekannte Wechselwirkungen zwischen diesen Allelen in der Gesamtbevölkerung und insbesondere in Patienten mit erhöhtem Risiko einer tiefen Venenthrombose aufzudecken.

In einer bevorzugten Ausführungsform des erfindungsgemäßen Verfahrens werden gleichzeitig alle genannten Polymorphismen mit Hilfe der DNA-Array-Technologie (wie in Beispiel 1 beschrieben, siehe unten) untersucht. In alternativen Ausführungsformen sind allerdings auch eine Vielzahl anderer Methoden anwendbar. Diese umfassen beispielsweise folgende Techniken:
1. Arrays auf Latex- oder anderen geeigneten Partikeln (sogenannten "soluble arrays" (62))
2. Methoden, basierend auf einer Polymerasekettenreaktion
   a. Multiplex-Polymerasekettenreaktion kombiniert mit allelspezifischer Hybridisierung auf Nylonfiltern (63).
   b. Multiplex-Polymerasekettenreaktion kombiniert mit allelspezifischer Hybridisierung in Multiwell-Platten oder -Streifen (z.B. Roche Diagnostics AG).
   c. Real-time Multiplex-Polymerasekettenreaktion unter Verwendung von Fluoreszenz-Markern (z.B. light cycler (64), Roche Diagnostics AG) oder anderen Markierungen.
3. Eine Mutiplex-Version der Ligasekettenreaktion (65).
4. Methoden, basierend auf einer DNA-Sequenzierung
   a. Konventionelle DNA-Sequenzierung unter Verwendung der Sanger didesoxy-Kettenabbruch-Reaktion (66).
   b. DNA-Sequenzierung mittels Matrix-unterstützter Laser Desorptions/-lonisierungs-Flugzeit-Massenspektrometrie (MALDI-TOF) (z.B. Sequenom technology (67) ).

Eine Vielzahl von Methoden kann herangezogen werden, um ein Zielmolekül auf einem Array oder einem anderen Substrat detektierbar zu machen. Dies schließt auch Methoden ein, die keine Markierung des Zielmoleküls erfordern, wie beispielsweise die Oberflächen-Plasmon-Resonanz Spektroskopie (68); eine direkte elektrische Messung (US-Patent: US 5,605,662), Oberflächenwellen-Sensoren (surface acoustic wave (SAW) sensors (69)), "Crystal-Microbalance-Dissipation" und verschiedene interferometrische Methoden sowie die reflektometrische Interferenz-Spektroskopie (70).

In einer bevorzugten Ausführungsform der Erfindung erfolgt die Markierung und Detektion der Zielmoleküle mittels Fluoreszenz und Gold/Silberfärbung (siehe Beispiel 1 unten). Neben einer einfachen Fluorszenzmarkierung können auch fluorszenzmarkierte DNA-Dendrimere genutzt werden, um die Sensitivität zu erhöhen (71). Ein weitere Alternative ist die Verwendung von Massenmarkierungen, die mittels Massenspetrometrie detektiert werden können (72). Anorganische Fluorszenzmarkierungen, die nicht wie organische Farbstoffe die Eigenschaft des Ausbleichens haben, können ebenfalls verwendet werden, wie up-converting phosphor (73) und quantum dots (74).

Außerdem können Chemilumineszenz und Biolumineszenz für die Markierung und Detektion zum Einsatz kommen. Diese Systeme basieren beispielsweise auf der durch Peroxidase aus Meerrettich katalysierten Umwandlung von Luminol und Wasserstoffperoxid zu einer chemiluminiszierenden Substanz oder auf der Verwendung von kommerziellen 1 1,2-Dioxetan-basierten Substraten wie CDP-Star®, AMPPD®, oder CSPD®.

Ebenso sind indirekte Markierungsverfahren für die Detektion eines Zielmoleküls möglich. Diese schließt das Biotin/Streptavidin-Kopplungssystem ein sowie Kopplungssysteme, die auf gekoppelten Enzymen oder gekoppelten Antikörpern basieren, die gegen kleine Moleküle wie Digoxigenin oder Fluorescein-lsothiocyanat gerichtet sind, die an die Ziel-DNA gekoppelt vorliegen.

Die vorliegende Erfindung betrifft zudem molekularbiologische Mittel zur Bestimmung genetischer Polymorphismen, die mit dem genetischen Risiko einer Thrombolie korrelieren. Diese können in einem diagnostischen Kit zusammengefaßt sein.

In einer bevorzugten Ausführungsform des erfindungsgemäßen Verfahrens werden Oligonukleotidprimer unter Reaktionsbedingungen eingesetzt, die die gleichzeitige Amplifikation der folgenden genetischen Polymorphismen ermöglichen: Faktor V^{Leiden}, Faktor V^{Cambridge}, die G20210A-Mutation im Prothrombin (Faktor ll)-Gen, den 4G/5G-Polymorphismus im Promoter des Plasminogen-Aktivator-Inhibitor-Gens, der C677T- und A1298C-Polymorphismus im MTHFR-Gen, die 68 bp-Insertion an Nukleotid 844 im CBS-Gen und der A2756G-Polymorphismus im Methionin-Synthase-Gen (Tabelle 1).

Mit Hilfe des erfindungsgemäßen Verfahrens können Wildtyp- und mutanten Allele gleichzeitig und selektiv durch Hybridisierung auf einem DNA-Array nachgewiesen werden. Ein weiterer Aspekt betrifft die Detektion der Wildtyp- und mutanten Allele auf der Basis von Fluoreszenz oder Silberfärbung, was entweder durch die direkte Markierung der Oligonukleotidproben oder durch indirekte Markierung, beispielsweise durch das Biotin/Streptavidin-Kopplungssystem ermöglicht werden kann.

### Definitionen

Im Rahmen dieser Erfindung umfasst der Begriff "Nukleinsäure" sowohl RNA als auch DNA (einschließlich deren Derivate), einschließlich cDNA, genomischer DNA und chemisch synthetisierte DNA genauso wie rekombinante, isolierte oder anderweitig modifizierte Nukleinsäuren (einschließlich chimärer Nukleinsäuren). Die Nukleinsäure kann einsträngig oder doppelsträngig sein. Wenn einzelsträngig, kann es sich bei der Nukleinsäure um den kodierenden oder den nicht-kodierenden Strang handeln. Außerdem kann die Nukleinsäure zirkulär oder linear sein.

Allgemein bezieht sich die PCR auf eine Methode oder Technik, in der die Zielnukleinsäure in zyklischen Polymerasereaktionen amplifiziert wird, wie es im US Patent 4,683,195 beschrieben wird. Diese Technik schließt ebenfalls Modifikationen ein, die auf der dort beschriebenen Methode aufbauen.

Der Begriff "Primer" bezieht sich auf Nukleinsäuren, die mit DNA hybridisieren und dabei ein freies 3'-Ende als Startpunkt für die Polymeraseaktivität bereitstellen.

Der Begriff "Polymorphismus" bezieht sich auf die Existenz verschiedener Allel eines Genes innerhalb einer Population. Diese Polymorphismen können - in heterozygotem oder homozygotem Status - Veränderungen im Phänotyp hervorrufen.

### Beispiel 1: Erfindungsgemäße Detektion genetischer Polymorphismen, die das Risiko einer Thromboembolie betreffen, mit Hilfe der DNA- Array-Technologie

### Ausführungsbeispiel 1: Gleichzeitige Detektion mittels Array-Tube-Technologie

In diesem Beispiel wird die Detektion relevanter Mutationen auf Basis der DNA-Array-Technologie durchgeführt, insbesondere unter Verwendung des Reaktionsgefäßes, des in der deutschen Patentanmeldung 102 01 463.9, "Reaktionsgefäß zur Durchführung von Array-Verfahren", (ClonDiag Chip Technologies GmbH) vom 16. Januar 2002. Die Technik zur Detektion der Interaktion der Proben mit dem Array ist Gegenstand der internationalen Patentanmeldung PCT/EP01/07575 "Verfahren zum qualitativen und/oder quantitativen Nachweis von molekularen Wechselwirkungen auf Sonden-Arrays", (ClonDiag Chip Technologies GmbH) vom 2. Juli 2001.

Die untersuchten Allele sind in Tabelle 1 aufgeführt.

**Tabelle 1:**

| Allele für den Thrombophilie-Chip | | | | | | | |
|---|---|---|---|---|---|---|---|
| **Position auf dem Array** | **Nukleotid** | | | **Aminosäure** | | | **Beschreibung** |
| | **Wildtyp** | **Position** | **Mutante** | **Wildtyp** | **Kodon** | **Mutante** | |
| 1 | G | 1691 | A | Arg | 506 | Gln | Faktor V^{Leiden} |
| 2 | G | 1091 | C | Arg | 306 | Thr | Faktor V^{Cambridge} |
| 3 | G | 20210 | A | | | | Faktor II (Prothrombin) Promoter |
| 4 | C | 677 | T | Ala | 222 | Val | Thermolabile Methylen-Tetrahydrofolat-Reduktase |
| 5 | A | 1298 | C | Glu | 428 | Ala | Thermolabile MethylenTetrahydrofolat-Reduktase |
| 6 | | 844 | ins 68 bp | | | | Cystathionin-beta-Synthase |
| 7 | 4G | Promoter | 5G | | | | Plasminogen-Aktivator-Inhibitor |
| 8 | A | 2756 | G | Asp | 919 | Gly | Methionin-Synthase |

### MATERIAL UND METHODEN

### A. Liste der verwendeten Puffer

Die Tabelle 2 führt die verwendeten Puffer auf.

**Tabelle 2:**

| Puffer | |
|---|---|
| **Puffer** | **Zusammensetzung** |
| 6 × SSPE/ 0,1% SDS-Puffer | 900 mM Natriumchlorid, 60 mM Natriumdihydrogenphosphat, 6 mM Ethylendiamintetraacetat, 0,1 % (w/v) Natriumdodecylsulfat, pH 7.4 |
| 3DNA-Puffer | 0.25 M Natriumphosphat, 1× Natriumchlorid/Natriumcitrat-Puffer (SSC), 4.5% (w/v) Natriumdodecylsulfat (SDS), 1 mM Ethylendiamintetraacetat (EDTA), pH 7.2 |
| 2 × MES (2-(N-morpholino) ethan sulfonsäure) | 200 mM MES, 1.8 M NaCI, 40 mM EDTA, 0.1 % (w/v) Cetyldimethylethylammoniumbromid, pH 6.8 |
| Waschlösung 1 | 2 × SSC, 0.1% SDS |
| Waschlösung 2 | 2 × SSC |
| Waschlösung 3 | 0.2 × SSC |

### B. Multiplex-PCR-Reaktion

### a. Primer

Um die Gensequenzen der in Tabelle 1 aufgelisteten Allele zu amplifizieren, wurde eine Multiplex-PCR-Reaktion für die gleichzeitige Amplifizierung von acht Sequenzen humaner DNA entwickelt. Die verwendeten Primer wurden von MWG Biotech (Ebersberg, Deutschland) synthetisiert und sind in Tabelle 3 aufgelistet.

Primer, die mit einem "s" bezeichnet sind, tragen an ihrem 5'-Ende entweder ein Biotinmolekül oder eine Cyanin 3.18- (Cy3) Fluoreszenzmarkierung. Die Qualifizierung mit "s" bedeutet dabei, daß es sich um Primer handelt, die komplementär zum Sence-Strang sind; während "as" auf die Komplementarität zum Anti-Sence-Strang hinweist.

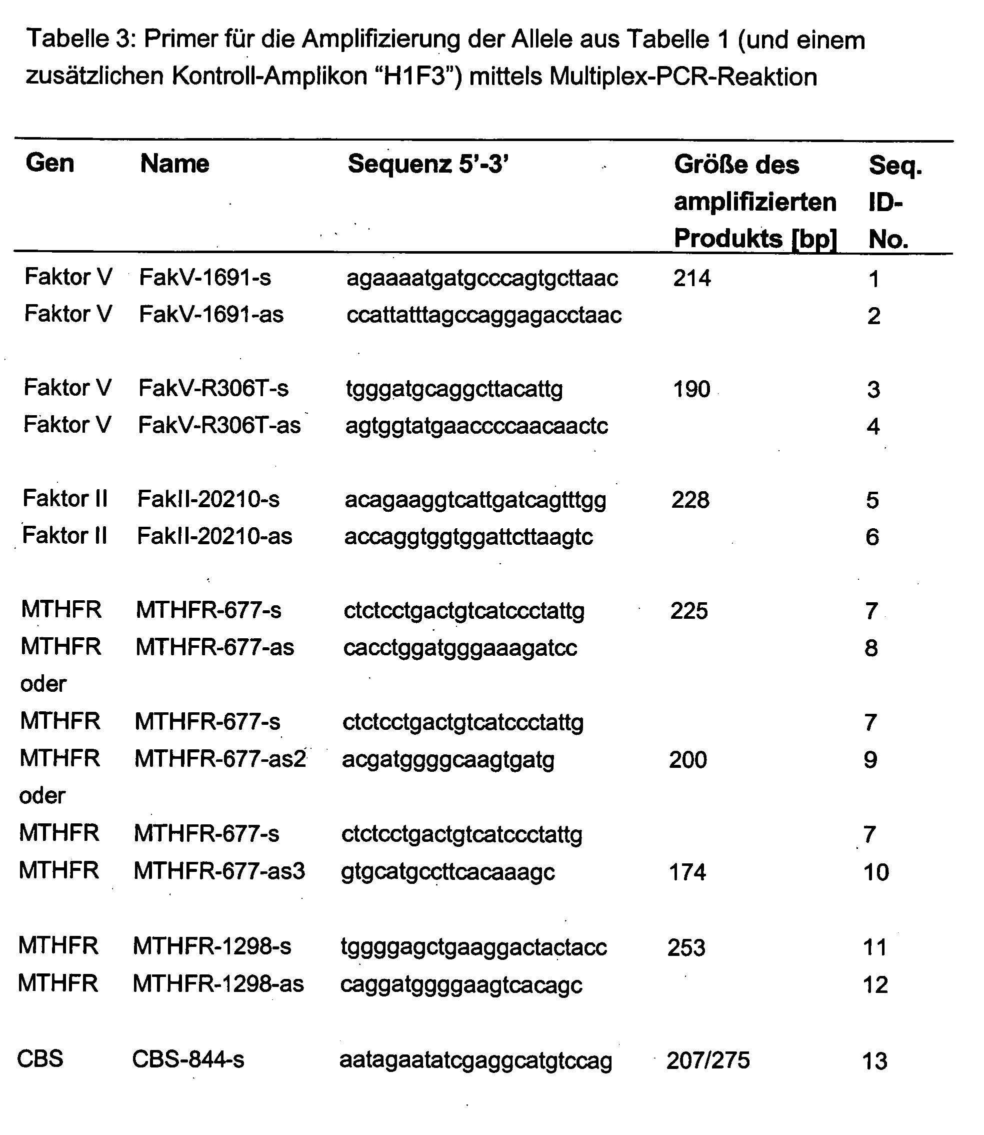

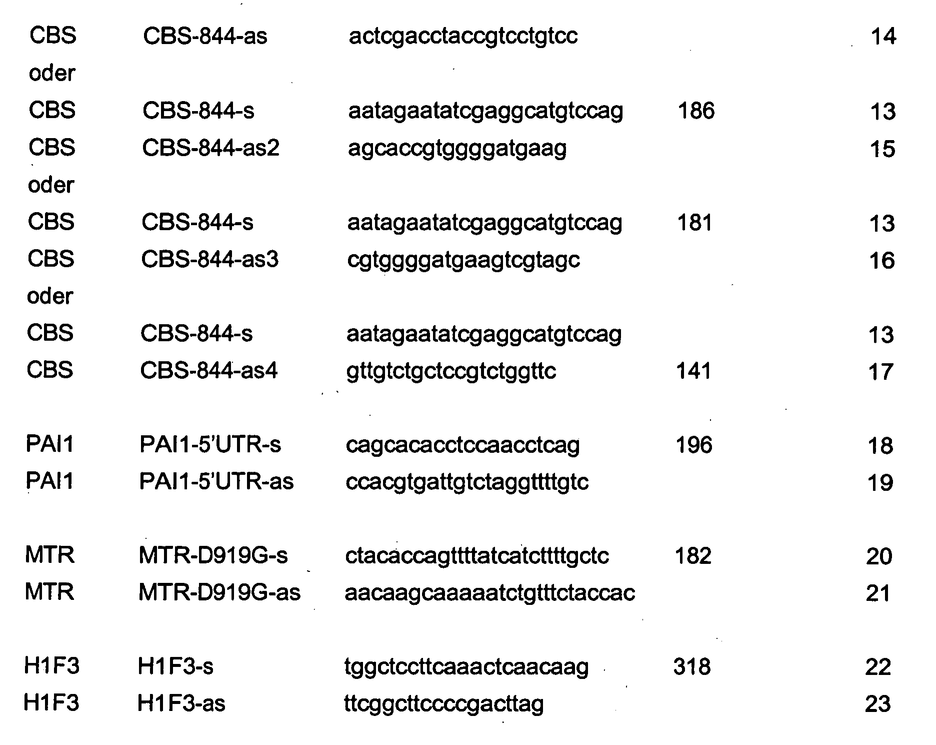

### b. Reaktionsmix für die Polymerasekettenreaktion

Die Zusammensetzung des Reaktionsmixes für die Polymerasekettenreaktion ist in Tabelle 4 wiedergegeben.

**Tabelle 4:**

| Reaktionsmix für 50 µl-PCR-Reaktionen. Die Angaben in Klammern geben die gesamte mögliche Breite des genannten Parameters an. Die Ansätze unter a) oder b) stellen Alternativen dar. | | |
|---|---|---|
| **a** | Jeder PCR-Primer | 400 nM (100 - 800 nM) |
| | humane genomische DNA | 100 ng (20 - 250 ng) |
| | dNTPs (jedes) | 250 µM (Amersham Biociences, Freiburg, Deutschland) (200 - 500 µM) |
| | HotStar-Taq DNA Polymerase | 5 U (Qiagen, Hilden, Deutschland) (2.5-10 U) |
| | 10× buffer | 5 µL (Qiagen) |
| oder | | |
| **b** | Jeder PCR-Primer | 800 nM (100 - 1200 nM) |
| | humane genomische DNA | 100 ng (20 - 250 ng) |
| | dNTPs (jedes) | 200 µM (Amersham Biociences) (200 - 500 µM) |
| | AccuPrime-Taq DNA Polymerase 10× Puffer | 10 U (Invitrogen, Paisley, GB) (2.5 - 20 U) 5 µL (Invitrogen) |

Die Multiplex-PCR-Reaktionen nach a) oder b) werden nach dem folgenden Protokoll in einem Perkin-Elmer 9600 Thermocycler (PE Biosystems, Langen, Deutschland) durchgeführt: Denaturierung, ein Zyklus bei 95 °C (92 bis 95°C) für 15 min (1-15 min); Amplifizierung in 35 Zyklen (20-45 Zyklen) mit Denaturierung bei 94°C (92-95°C) für 1 min, Annealing bei 56°C (52 bis 62°C) für 2 min (2-4 min) und Verlängerung bei 68°C (66-72°C) für 1 min (0-10min); anschließende Verlängerung, ein Zyklus bei 68°C (60-72°C) für 5 min (5-30 min). Die relative Ausbeute und die Größe der einzelnen Fragmente wird mittels Kapillarelektrophorese in einem ABI Prism 310C oder einem ABI Prism 3100 Avant DNA-Sequenziergerät (Applied Biosystems, Weiterstadt, Deutschland) bestimmt. Altemativ zu den Multiplex-PCR-Methoden a) und b) wurde das Multiplex PCR-Kit der Firma Qiagen (Hilden, Deutschland), in Verbindung mit jeweils 100 bis 1200 nM PCR-Primer und 20 bis 250 ng humaner, genomischer DNA und den im vorhergehenden Absatz beschriebenen Amplifikationsschritten verwendet.

### C. Herstellung von DNA Test-Arrays in Array-Tubes

Die PCR-Produkte werden mit immobilisierten DNA-Proben in Reaktionsgefäßen (Array-Tubes) hybridsiert, wie in der deutschen Patentanmeldung 102 01 463.9, "Reaktionsgefäß zur Durchführung von Array-Verfahren" beschrieben (siehe oben). Die Technik zur Detektion der Interaktion der PCR-Produkte mit dem Array ist Gegenstand der internationalen Patentanmeldung PCT/EP01/07575 "Verfahren zum qualitativen und/oder quantitativen Nachweis von molekularen Wechselwirkungen auf Sonden-Arrays" (siehe oben). Die Sonden auf dem Array bestehen aus synthetischer einzelsträngiger DNA mit kovalent gebundenem Aminolink am 5'-Ende (MWG Biotech). Alle verwendeten Sonden sind in der Tabelle 5 aufgeführt.

Die Sonden wurden so ausgesucht, daß sie entweder die Wildtypsequenz oder die an der Position des Polymorphismus entsprechend veränderte Sequenz repräsentieren. Die Sonden werden auf das Array aufgebracht, indem entweder herkömmliche Spotting-Verfahren mit einem MicroGrid II Microarray Spotter (Biorobotics Ltd., Cambridge, GB) auf Glasobjektträgem mit Epoxy-modifizierter Oberfläche verwendet werden oder eine *in situ*-Synthese durchgeführt wird, wie sie auf Seite 61 der deutschen Patentanmeldung 102 01 463.9 (siehe oben). Die Sonden werden in einer Lösung aus 0.1 M Na₃PO₄, 2.2 % Na₂SO₄, pH 9.0 aufgebracht. Danach werden die Amino-modifizierten Sonden durch 30-minütiges Backen bei 60°C kovalent an die Epoxygruppen der Objektträgeroberfläche gebunden.

Die Mittelpunkte benachbarter Spots haben einen Abstand von 200 µm, die gesamte Fläche eines Arrays beträgt 2,4 x 2,4 mm. Auf diese Weise können mehr als 100 identische Arrays auf einem einzigen Objektträger der Größe 75 x 25 mm erstellt werden. Ein einzelnes Array besteht dabei aus 12 x 10 =120 Spotpositionen.

Die Spots der äußersten Reihe auf jeder Seite des Arrays werden für Kontrollen der Silberfärbung genutzt, so daß 100 Spots übrigbleiben, die folgendermaßen genutzt werden:
1. Zur Alleldetektion; wie oben beschrieben. (Um die Effizienz der Hybridisierungsreaktion zu überwachen, wird jede Sonde doppelt oder dreifach gespottet).
2. Zur Kontrolle der PCR-Effizienz (Jede Sonde wird üblicherweise doppelt gespottet).
3. Zur Kontrolle der Hybridisierungstemperatur.

### D. Kontrollspots für die DNA-Test-Arrays in Array-Tubes

Zusätzlich zu den Sonden, die zur Detektion der genetischen Varianten genutzt werden, weist das Array Kontrollspots auf, die zur Qualitätskontrolle des Spotting und als Markierungen für die Orientierung des Arrays bei der Auswertung dienen. Im einzelnen enthält der Array folgende vier Typen von Kontrollspots:
a. Kontrolle der Silberfärbung
   Zwei verschiedene Oligonukleotide (Bio-P1 cctctgcagactactattac und Bio-P1' gtaatagtagtctgcagagg), die eine 5'-Aminomodifizierung und eine 3'-Biotinmarkierung aufweisen, werden in äquimolarem Verhältnis gemischt und an sieben Positionen in den beiden äußersten Reihen des Arrays gespottet. Diese Sonden dienen als Kontrolle für die Silberfärbungsreaktion. Außerdem werden diese Spots genutzt, um das Array bei der Auswertung im Array-Lesegerät korrekt auszurichten.
b. Kontrolle der Effizienz der Polymerasekettenreaktion
   Mit Hilfe amplikonspezifischer Sonden wird überprüft, ob ausreichende Mengen der acht PCR-Produkte gebildet werden. Die Sequenzen dieser Sonden unterscheiden sich von denjenigen, die zur Detektion der Polymorphismen eingesetzt werden und enthalten keine bekannten Mutationen. Die verwendeten Oligonukleotide wurden so ausgesucht, daß ihre theoretische Schmelztemperatur 5 bis 8°C über derjenigen der Sonden liegt, die zur Detektion der Polymorphismen eingesetzt werden. Die verwendeten Sonden sind in Tabelle 6 aufgeführt.
c. Kontrolle der Arrayqualität
   Der Spottingpuffer wird wir oben beschrieben zusammengesetzt. Er erlaubt eine Visualisierung der Spots vor der Hybridisierung. Dies ermöglicht es, die Qualität und Morphologie der Spots jedes Arrays im Array-Lesegerät vor der Hybridisierung zu beurteilen. Nicht gewaschene Array-Tubes werden im Dunkeln bei Raumtemperatur gelagert. Nach bis zu dreimonatiger Lagerung ist keine detektierbare Verringerung der Hybridisierungssignale festzustellen und die Array-Tubes können direkt ohne weitere Behandlung verwendet werden.
d. Kontrolle der Hybridisierungsbedingungen
   Die Verwendung der geeigneten Hybridiserungstemperatur wird ermittelt, indem zwei der Sonden verwendet werden, die in Tabelle 7 aufgeführt sind.

Wenn die Hybridisierungstemperatur zu niedrig war, geben beide Sonden ein Hybridisierungssignal, war die Temperatur zu hoch, reagiert keine der beiden positiv. Die gewünschte Hybridisierungstemperatur gibt nur ein Signal und zwar bei dem Spot, der die längere Sonde enthält. Ein zusätzliches PCR-Produkt, das eine Sequenz des hochkonservierten Histons H1F3 enthält, dient als Probe für diese Sonden. Die korrespondierenden PCR-Primer (H1F3-s tggctccttcaaactcaacaag and H1F3-as ttcggcttccccgacttag) werden in die Multiplex-PCR-Reaktion integriert. Das resultierende PCR-Produkt besteht aus 318 bp.

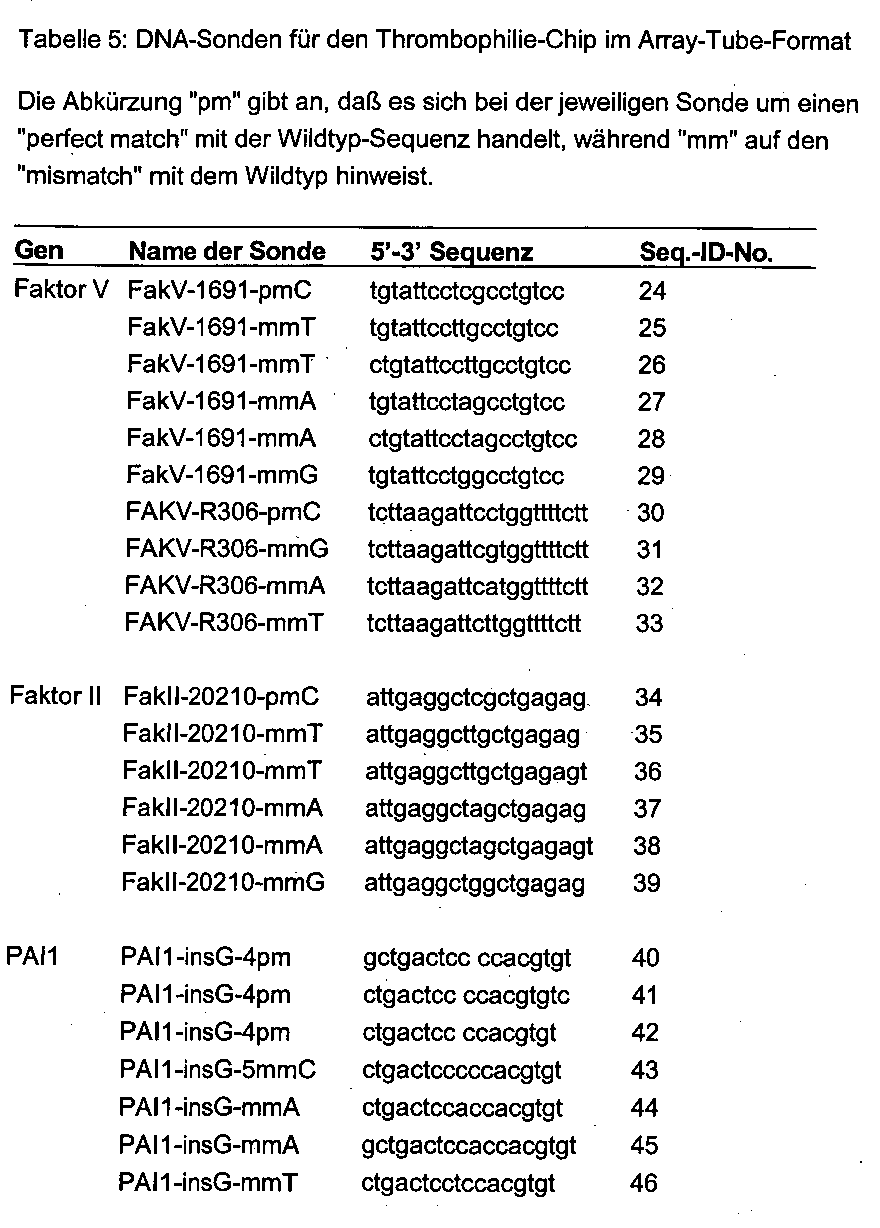

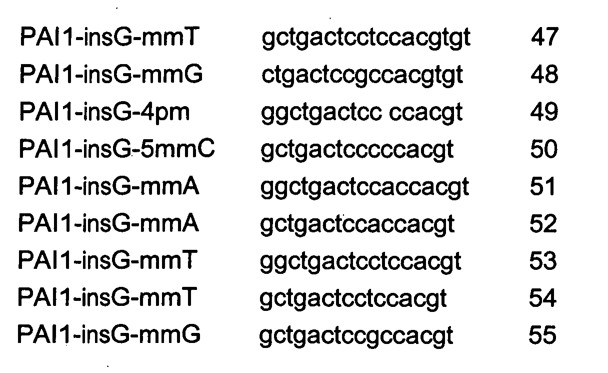

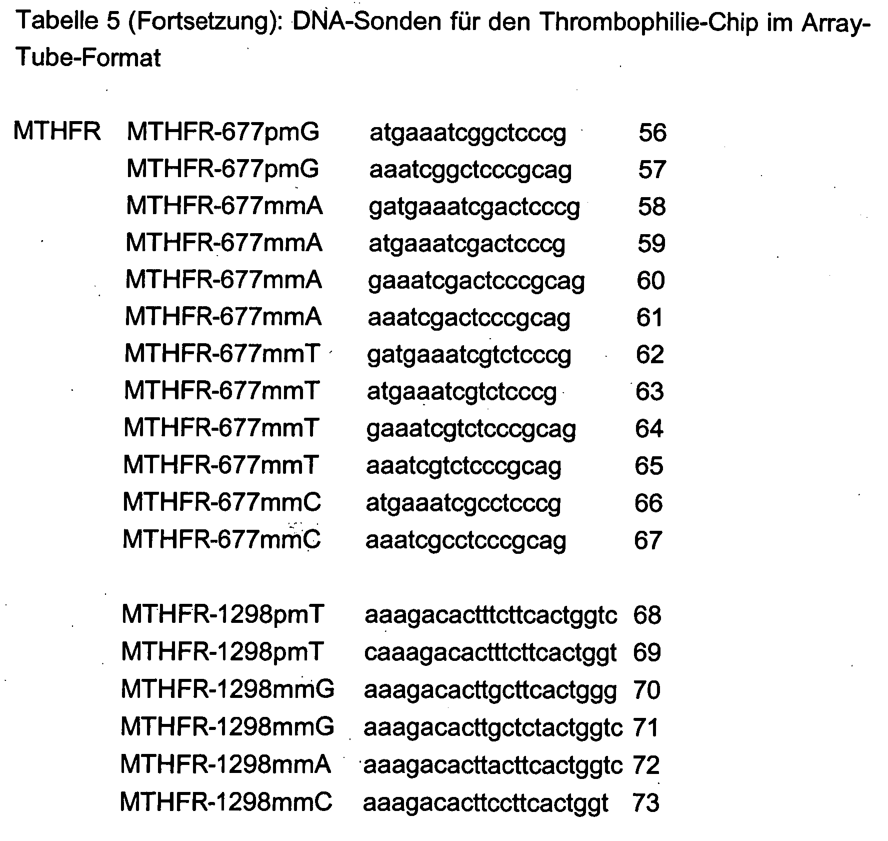

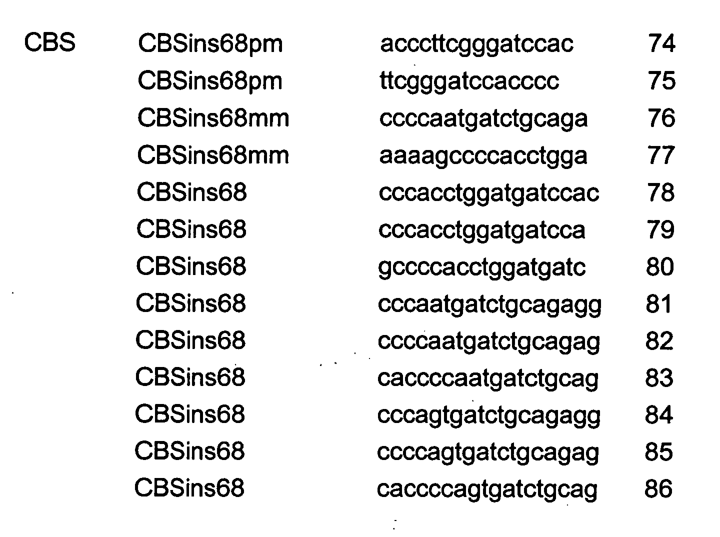

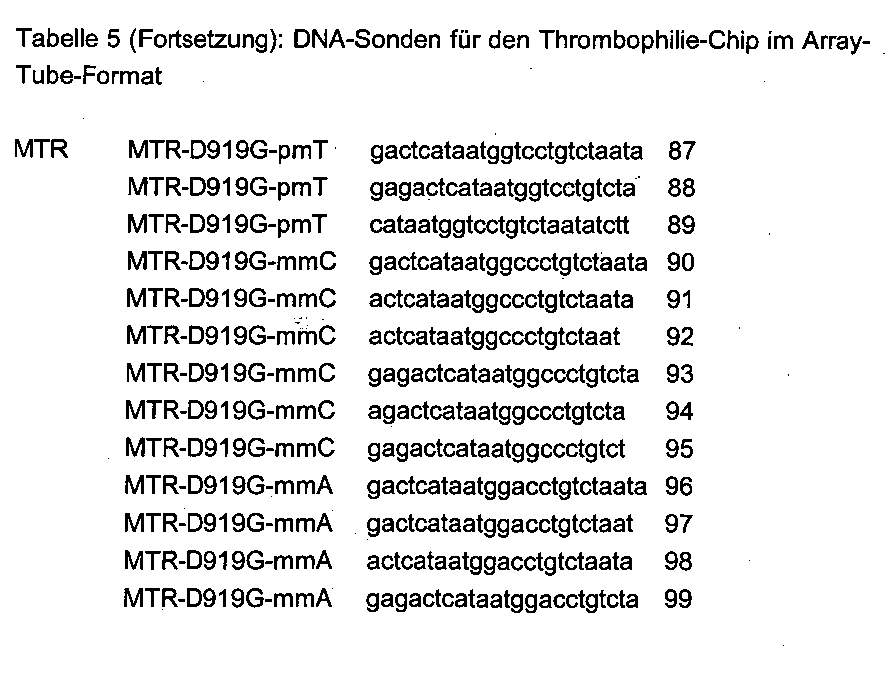

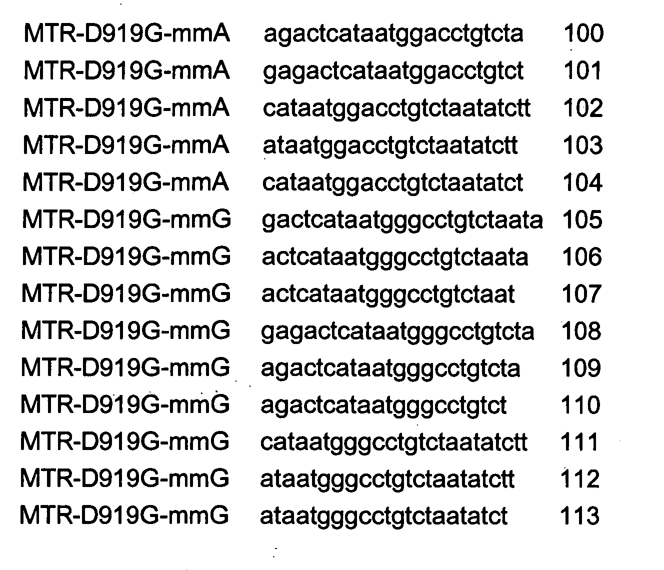

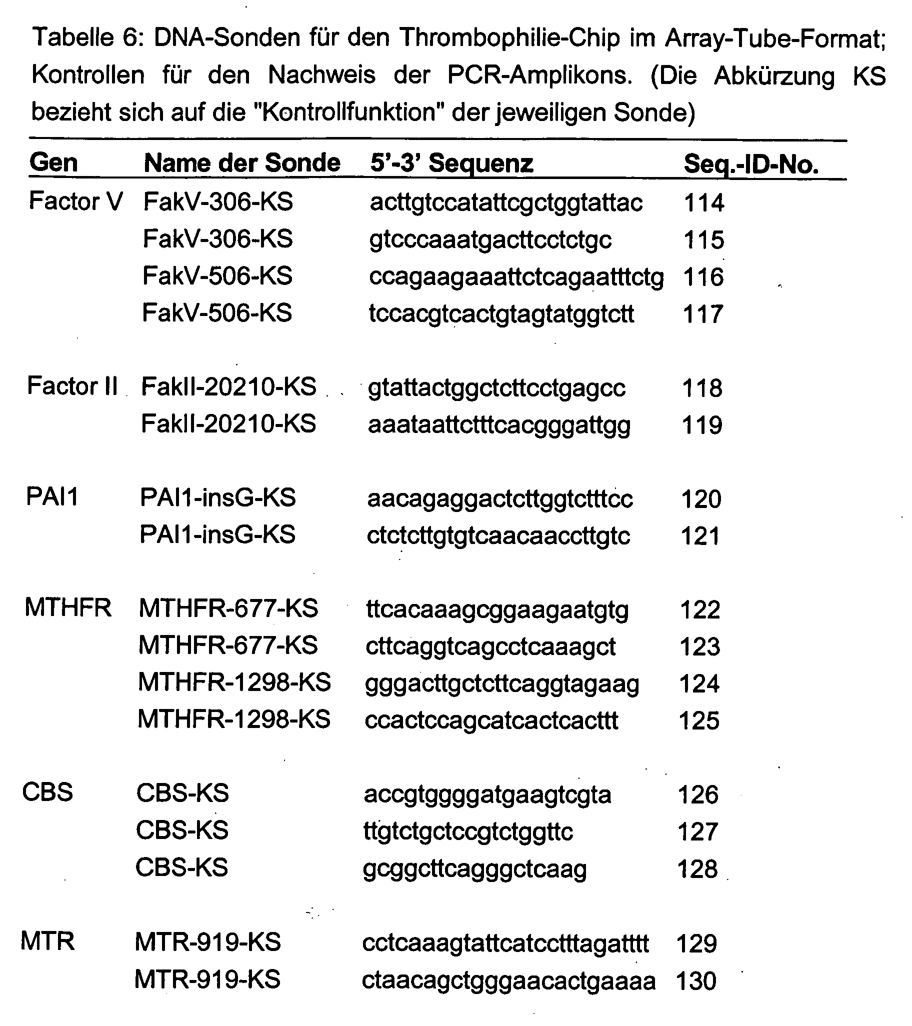

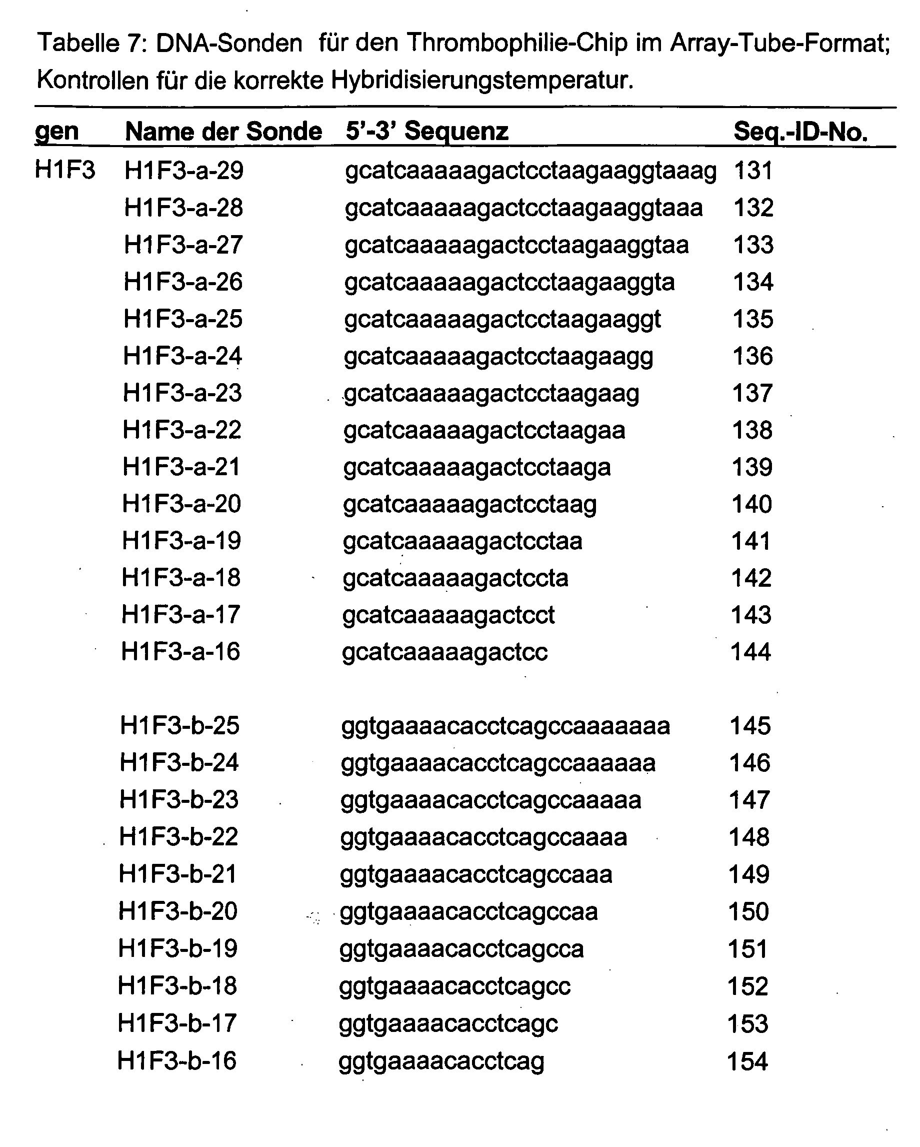

### E. Hybridisierung von DNA-Arrays in Array-Tubes

Nachdem die Sonden auf die Obejektträgeroberfläche aufgebracht worden sind, werden die Objektträger in einer feuchten Kammer bei 37°C für 30 min inkubiert. Die PCR-Primer, die verwendet werden, um die Proben für die Hybridisierung auf dem DNA-Array zu generieren, sind an ihrem 5'-Ende kovalent mit Biotin markiert.

Vor der Hybridisierung des PCR-Reaktionsansatzes im Array-Tube werden 150 µl Hybridisierungslösung hergestellt, indem 5 bis 50 µl des PCR-Reaktionsansatzes mit 145 bis 100 µl des Hybridisierungspuffers (3DNA-Puffer, 2 × MES Puffer oder 6 x SSPE/ 0,1% SDS-Puffer; Tabelle 2) versetzt und 4 min bei 98°C inkubiert werden. Die Hybridisierungslösung wird in das Array-Tube gegeben und für 90 min bei 30°C und 350 Umdrehungen pro Minute (Upm) in einem Thermoschüttler (Eppendorf, Hamburg, Deutschland) inkubiert. Die Hybridisierungslösung wird abgesaugt und 500 µl, auf 55°C vorgewärmter Hybridisierungspuffer (3DNA-Puffer, 2 × MES Puffer oder 6 x SSPE/ 0,1% SDS-Puffer) zugegeben und bei 55°C und 350 Upm für 10 min inkubiert. Dann wird der Inhalt des Array-Tubes abgesaugt und durch 500 µl Waschlösung 1 ersetzt, mit der das Array-Tube 10 min bei 30°C und 750 Upm inkubiert wird. Das Array-Tube wird wieder leergesaugt und 500 µl Waschlösung 2 zugegeben und 10 min bei 20°C und 750 Upm inkubiert. Wieder wird das Array-Tube leergesaugt und das Array mit 150 µl Blockierungslösung, die aus 2% entfettetem Milchpulver ("Lasana" oder "Magermilchpulver", Applichem, Darmstadt, Deutschland) in Hybridisierungspuffer (3DNA-Puffer, 2 × MES Puffer oder 6 x SSPE/ 0,1% SDS-Puffer) besteht, für 15 min bei 30°C und 350 Upm inkubiert. Das Array-Tube wird leergesaugt und 150 µl Streptavidin-Gold-Konjugat-Lösung (Goldpartikel: 5 bzw. 6 nm; British Biocell Ltd., Cardiff, GB oder Aurion N.V., Wageningen, NL) zugegeben und für 15 min bei 30°C und 350 Upm inkubiert. Dann wird der Inhalt des Array-Tubes abgesaugt und durch 500 µl Waschlösung 1 ersetzt, mit der das Array-Tube 10 min bei 30°C und 750 Upm inkubiert wird. Das Array-Tube wird wieder leergesaugt und 500 µl Waschlösung 2 zugegeben und 10 min bei 20°C und 750 Upm inkubiert. Das Array-Tube wird nun zum letzten Mal leergesaugt und gewaschen, indem 500 µl Waschlösung 3 zugesetzt werden und für 10 min bei 20°C und 750 Upm inkubiert wird.

### F. Entwicklung des Array-Tubes

Das Array-Tube-Lesegerät (AT-01, ClonDiag GmbH, Jena bzw. seine Folgeprodukte) wird auf seine Betriebstemperatur von 25 °C vorgeheizt und der Grauwert eingestellt. Die Entwicklungslösung (British Biocell, UK oder Aurion, NL) wird präpariert, indem gleiche Volumina von "Initiator"- bzw. "Developer"- und "Enhancer"-Lösung gemischt und für 1 min bei 25°C inkubiert werden. 150 µl dieser Entwicklungslösung werden in das Array-Tube gegeben. Die Entwicklung der Silberfärbung wird beobachtet, indem während eines Zeitraumes von 40 min Bilder des Arrays in 20 sec-Intervallen im Array-Tube-Lesegerät aufgenommen werden.

### G. Auswertung des Array-Tubes

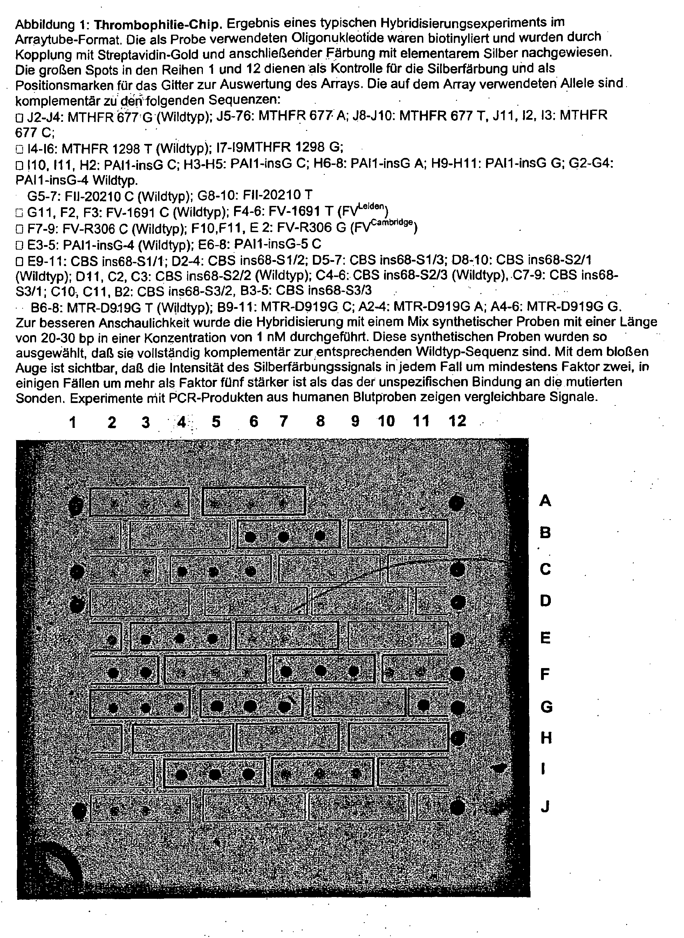

Für jede Sonde auf dem DNA-Array wird die Intensität der Silberfärbungsreaktion ermittelt, wie es in der internationalen Patentanmeldung PCT/EP01/07575 (siehe oben) beschrieben ist, das bereits weiter oben zitiert wurde. Basierend auf der relativen Signalintensität für jede Sonde, wird der Status der untersuchten Probe als homozygot Wildtyp (bei ausschließlicher Hybridisierung mit der Sonde mit zum Wildtyp komplementärer Sequenz), homozygot variant (bei ausschließlicher Hybridisierung mit der Sonde mit zum Polymorphismus komplementärer Sequenz) oder als heterozygot (bei Hybridisierung mit beiden, der Wildtyp-spezifischen und der Polymorphismus-spezifischen, Sonde, aber nicht mit den übrigen Sonden des Arrays). Ein typisches Ergebnis einer solchen Hybridisierung im Array-Tube-Format ist in Abbildung 1 dargestellt.

### Ausführungsbeispiel 2: Detektion genetischer Polymorphismen mit Hilfe "gespotteter DNA-Arrays auf Glasobjekträgern"

### MATERIAL UND METHODEN

### A. Liste der verwendeten Puffer

Die Puffer, sind in Tabelle 2 aufgeführt.

### B. Multiplex-PCR-Reaktion

In der PCR-Reaktion zur Amplifizierung der Gensequenzen aller Allele, der Tabelle 1, werden die Primer aus der Tabelle 3 verwendet. Der PCR-Reaktionsmix entspricht den Angaben aus der Tabelle 4. Die Durchführungsbedingungen für die Multiplex-PCR-Reaktion sind im Abschnitt "b. Reaktionsmix für die Polymerasekettenreaktion" dargestellt.

### C. Herstellung von DNA Test-Arrays auf Glasobjektträgern

Die Sonden auf dem Array bestehen aus synthetischer einzelsträngiger DNA mit kovalent gebundenem Aminolink am 5'-Ende (MWG Biotech). Die Sonden sind in Tabelle 4 aufgeführt. Die Sonden wurden so ausgesucht, daß sie alle an der Position des Polymorphismus möglichen vier Nukleotide repräsentieren. Jede Sonde wird üblicherweise als Block von fünf benachbarten Spots auf dem Array angeordnet. Die Sonden werden auf das Array aufgebracht, indem herkömmliche Spotting-Verfahren mit einem MicroGrid II Microarray Spotter (Biorobotics Ltd., Cambridge, GB) auf Glasobjektträgern mit Epoxy-modifizierter Oberfläche verwendet werden.

Die Sonden werden als 10µM-Lösung in einem Puffer aus 150 mM Natriumphosphat, 0.005% (w/v) Natriumdodecylsulfat, pH 8.5 oder 50 mM Natriumphosphat, 0.0025 % (w/v) Natriumdodecylsulfat, pH 8.5 aufgebracht. Die gespotteten Objektträger werden bei 60°C für 30 min gebacken, um die Amino-modifizierten Sonden kovalent an die Epoxygruppen auf der Glasoberfläche zu binden. Die hergestellten Spots haben einen Durchmesser von circa 150 µm, die Mittelpunkte benachbarter Spots haben einen Abstand von 400 µm.

### D. Kontrollspots für die DNA-Test-Arrays auf Glasobjektträgern

Zusätzlich zu den Sonden, die zur Detektion der genetischen Varianten genutzt werden, weist das Array Kontrollspots auf, die zur Qualitätskontrolle des Spotting und als Markierungen für die Orientierung des Arrays bei der Auswertung dienen. Im einzelnen enthält der Array folgende vier Typen von Kontrollspots:
a. Kontrolle des Fluoreszenzsignals
   Ein Oligonukleotid (Cy3-Sonde, 5'-agagagagagag-3'), das eine 5'-Amino-Modifizierung und eine 3'-Cy3-Markierung aufweist, wird an fünf bis zehn randständigen Positionen des Arrays gespottet. Diese Spots dienen als Kontrolle für die Fluoreszenzdetektion. Das entstehende Spotmuster wird außerdem genutzt, um die Orientierung des Arrays im Lesegerät zu bestimmen.
b. Kontrolle der Effizienz der Polymerasekettenreaktion
   Mit Hilfe amplikonspezifischer Sonden wird überprüft, ob ausreichende Mengen der acht PCR-Produkte gebildet wurden. Die Sequenzen dieser Sonden unterscheiden sich von denjenigen, die zur Detektion der Polymorphismen eingesetzt werden und enthalten keine bekannten Mutationen. Die verwendeten Oligonukleotide wurden so ausgesucht, daß ihre theoretische Schmelztemperatur 5 bis 8°C über derjenigen der Sonden liegt, die zur Detektion der Polymorphismen eingesetzt werden. Die verwendeten Sonden sind in Tabelle 6 aufgeführt.
c. Kontrolle der Arrayqualität auf dem Glasobjektträger
   Jeder gespottete Slide wird mit der höchstmöglichen Verstärkungsrate im Affimetrix ArrayScanner 428 (Affymetrix Inc., Santa Clara, CA, USA) untersucht. Unter diesen Bedingungen ist die Autofluoreszenz der DNA ausreichend, um die Spots sichtbar zu machen und somit ihre Qualität und Morphologie zu beurteilen. Außerdem ist jeder zehnte gespottete Objektträger ein "QMT VisArray Slide" (Quantifoil, Jena, Deutschland), der Spots als dunkelblaue Punkte vor gelbem Hintergrund sichtbar macht.
d. Kontrolle der Hybridisierungsbedingungen
   Die Verwendung der korrekten Hybridiserungstemperatur wird ermittelt, indem zwei der Sonden, aus der Tabelle 7, in derselben Weise verwendet werden, wie es unter der Überschrift "Kontrolle der Hybridisierungsbedingungen" im Abschnitt "D. Kontrollspots für die DNA-Test-Arrays in Array-Tubes" beschrieben wird.

### E. Hybridisierung von DNA-Arrays auf Glasobjektträgern

HybriWells (Schleicher & Schuell GmbH, Dassel, Deutschland) werden auf die Objektträger geklebt. Anschließend werden die Objektträger in die "Super-Inkubator 3002"-lnkubationskammern (Ogham GmbH, Münster, Deutschland) eingebracht, die vorher im Thermomixer, versehen mit MTP-Block (Eppendorf GmbH, Eppendorf, Deutschland), auf 30°C vorgeheizt wurden. Die Hybridisierungslösung wird hergestellt, indem 50 µl des PCR-Reaktionsansatzes mit 200 µl des 3DNA-Puffer (Tabelle 2) versetzt und 4 min bei 98°C inkubiert werden. Die Hybridisierunglösung wird in das HybriWell gegeben, das anschließend verschlossen wird. Der so behandelte Objektträger wird für 90 min bei 30°C inkubiert, um den PCR-Produkten die Hybridisierung mit den gespotteten DNA-Sonden auf dem Array zu ermöglichen. Nach der Hybridisierung wird das HybriWell vom Objektträger entfernt und die Objektträger werden, paarweise mit der gespotteten Oberfläche nach außen, für 10 min in ein Färbegefäß mit 100 ml, auf 55°C vorgeheizten, 3DNA-Puffer inkubiert. Die Objektträger werden anschließend dann einmal für 10 min bei 30°C in Waschlösung 1, einmal bei Raumtemperatur in Waschlösung 2 und einmal bei Raumtemperatur in Waschlösung 3 inkubiert und anschließend im Stickstoffstrom oder einer Objektträgerzentrifuge (TeleChem Ltd., Sunnyvale, CA, USA) getrocknet.

### F. Detektion von DNA-Arrays auf Glasobjektträgern

Die Detektion der Fluorszenzsignale erfolgt unter Verwendung des Cyanin3 (Cy3)-Kanals eines Fluoreszenzscanners (Array Scanner 428, Affymetrix Inc., Santa Clara, CA, USA) bei 570 nm. Das gemessene Spotsignal, in diesem Fall das Cy3-Signal, ist dabei proportional zur Menge der an die korrespondierende Sonde gebundenen Zielmoleküle.

### G. Auswertung von DNA-Arrays auf Glasobjektträgern

Für jede Sonde auf dem DNA-Array wird die Intensität des Fluoreszenzsignals mit Hilfe der IconoClus Software (ClonDiag GmbH, Jena, Deutschland) ermittelt. Basierend auf der relativen Signalintensität für jede Sonde, wird der Status der untersuchten Probe als homozygot Wildtyp (bei ausschließlicher Hybridisierung mit der Sonde mit zum Wildtyp komplementärer Sequenz), homozygot variant (bei ausschließlicher Hybridisierung mit der Sonde mit zum Polymorphismus komplementärer Sequenz) oder als heterozygot (bei Hybridisierung mit beiden, der Wildtyp-spezifischen und der Polymorphismus-spezifischen, Sonde, aber nicht mit den übrigen Sonden des Arrays).

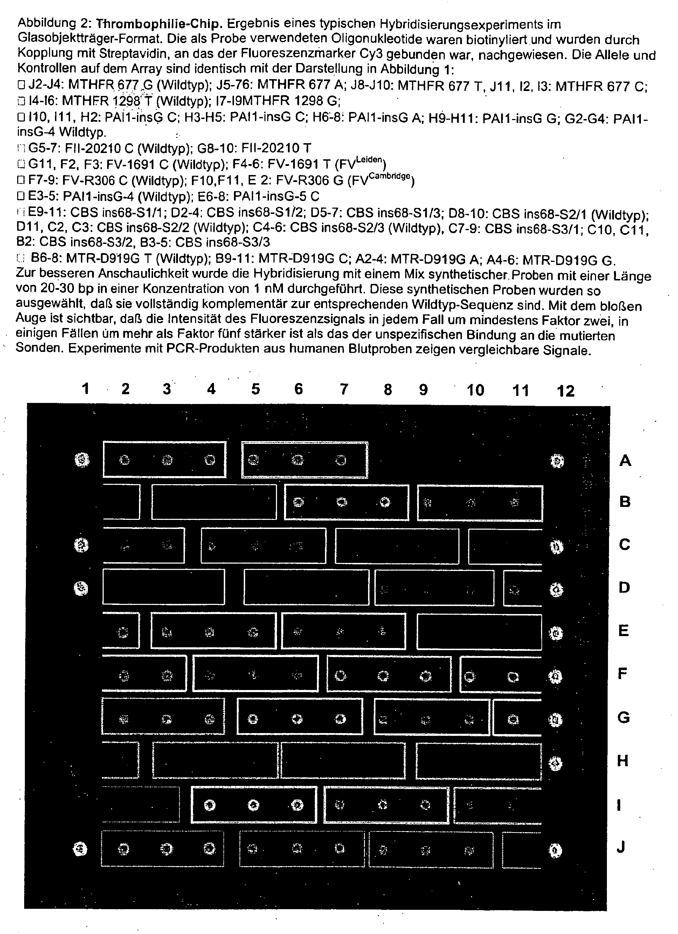

Ein typisches Ergebnis einer solchen Hybridisierung mit einem Glasobjektträger ist in Abbildung 2 dargestellt.

### Referenzliste

1. Osterud B, Rapaport Sl. Activation of factor IX by the reaction product of tissue factor and factor VII: additional pathway for initiating blood coagulation. *Proc Natl Acad Sci USA.* 1977;74:5260-5264.
2. Davie EW, Fujikawa K, Kisiel W. The coagulation cascade: initiation, maintenance, and regulation. *Biochemistry.* 1991;30:10363-10370.
3. Mann KG, Krishnaswamy S, Lawson JH. Surface-dependent hemostasis. *Semin Hematol.* 1992;29:213-226.
4. Gailani D, Broze GJ, Jr. Factor XI activation in a revised model of blood coagulation. *Science.* 1991;253:909-912.
5. Suzuki K, Stenflo J, Dahlbäck B, Teodorsson B. Inactivation of human coagulation factor V by activated protein C. *J Biol Chem.* 1983;258:1914-1920.
6. Solymoss S, Tucker MM, Tracy PB. Kinetics of inactivation of membrane-bound factor Va by activated protein C. Protein S modulates factor Xa protection. *J Biol Chem.* 1988;263:14884-14890.
7. Vehar GA, Davie EW. Preparation and properties of bovine factor VIII (antihemophilic factor). *Biochemistry.* 1980;19:401-410.
8. Marlar RA, Kleiss AJ, Griffin JH. Mechanism of action of human activated protein C, a thrombin-dependent anticoagulant enzyme. *Blood.* 1982;59:1067-1072.
9. Fulcher CA, Gardiner JE, Griffin JH, Zimmerman TS. Proteolytic inactivation of human factor VIII procoagulant protein by activated human protein C and its analogy with factor V. *Blood.* 1984;63:486-489.
10. Eaton D, Rodriguez H, Vehar GA. Proteolytic processing of human factor VIII. Correlation of specific cleavages by thrombin, factor Xa, and activated protein C with activation and inactivation of factor VIII coagulant activity. *Biochemistry.* 1986;25:505-512.
11. Koedam JA, Meijers JC, Sixma JJ, Bouma BN. Inactivation of human factor VIII by activated protein C. Cofactor activity of protein S and protective effect of von Willebrand factor. *J Clin Invest.* 1988:82:1236-1243.
12. Pittman DD, Kaufman RJ. Proteolytic requirements for thrombin activation of anti-hemophilic factor (factor VIII). *Proc Natl Acad Sci U S A.* 1988;85:2429-2433.
13. Fay PJ, Smudzin TM, Walker FJ. Activated protein C-catalyzed inactivation of human factor VIII and factor Villa. Identification of cleavage sites and correlation of proteolysis with cofactor activity. *J Biol Chem.* 1991 ;266:20139-20145.
14. Bertina RM, Koeleman BP, Koster T, Rosendaal FR, Dirven RJ, de Ronde H, van der Velden PA, Reitsma PH. Mutation in blood coagulation factor V associated with resistance to activated protein C. *Nature.* 1994;369:64-67.
15. Dahlbäck B. Inherited thrombophilia: resistance to activated protein C as a pathogenic. factor of venous thromboembolism. *Blood.* 1995;85:607-614.
16. Dahlbäck B. Molecular genetics of venous thromboembolism. *Ann Med.* 1995;27:187-192.
17. Dahlbäck B. Molecular genetics of thrombophilia: factor V gene mutation causing resistance to activated protein C as a basis of the hypercoagulable state. *J Lab Clin Med.* 1995;125:566-571.
18. Dahlbäck B. Factor V gene mutation causing inherited resistance to activated protein C as a basis for venous thromboembolism. *J Intern Med.* 1995;237:221-227.
19. Vandenbroucke JP, Koster T, Briet E, Reitsma PH, Bertina RM, Rosendaal FR. Increased risk of venous thrombosis in oral-contraceptive users who are carriers of factor V Leiden mutation. *Lancet.* 1994;344:1453-1457.
20. Williamson D, Brown K, Luddington R, Baglin C, Baglin T. Factor V Cambridge: a new mutation (Arg306->Thr) associated with resistance to activated protein C. *Blood.* 1998:91:1140-1144.
21. Poort SR, Rosendaal FR, Reitsma PH, Bertina RM. A common genetic variation in the 3'-untranslated region of the prothrombin gene is associated with elevated plasma prothrombin levels and an increase in venous thrombosis. *Blood.* 1996;88:3698-3703.
22. Martinelli 1, Sacchi E, Landi G, Taioli E, Duca F, Mannucci PM. High risk of cerebral-vein thrombosis in carriers of a prothrombin-gene mutation and in users of oral contraceptives. *N Engl J Med.* 1998:338:1793-1797.
23. Martinelli l, Taioli E, Bucciarelli P, Akhavan S, Mannucci PM. Interaction between the G20210A mutation of the prothrombin gene and oral contraceptive use in deep vein thrombosis. *Arterioscler Thromb Vasc Biol.* 1999;19:700-703.
24. Aznar J, Vaya A, Estelles A et al. Risk of venous thrombosis in carriers of the prothrombin G20210A variant and factor V Leiden and their interaction with oral contraceptives. *Haematologica.* 2000;85:1271-1276.
25. Mira Y, Aznar J, Estelles A, Vaya A, Villa P, Ferrando F. Congenital and acquired thrombotic risk factors in women using oral contraceptives: clinical aspects. *Clin Appl Thromb Hemost.* 2000;6:162-168.
26. Emmerich J, Rosendaal FR, Cattaneo M et al. Combined effect of factor V Leiden and prothrombin 20210A on the risk of venous thromboembolism-pooled analysis of 8 case-control studies including 2310 cases and 3204 controls. Study Group for Pooled-Analysis in Venous Thromboembolism. *Thromb Haemost.* 2001;86:809-816.
27. Arruda VR, Annichino-Bizzacchi JM, Goncalves MS, Costa FF. Prevalence of the prothrombin gene variant (nt20210A) in venous thrombosis and arterial disease. *Thromb Haemost.* 1997;78:1430-1433.
28. Doggen CJ, Cats VM, Bertina RM, Rosendaal FR. Interaction of coagulation defects and cardiovascular risk factors: increased risk of myocardial infarction associated with factor V Leiden or prothrombin 20210A. *Circulation.* 1998;97:1037-1041.
29. Tripodi A, Mannucci PM. Laboratory investigation of thrombophilia. *Clin Chem.* 2001;47:1597-1606.
30. Witt l. Pathobiochemie und klinisch-chemische Diagnostik der Organund Systemerkrankungen: Hämostase- und Fibrinolysesystem. In: Greiling H, Gressner AM, editors. Lehrbuch der Klinischen Chemie und Pathobiochemie. Stuttgart: Schattauer Verlag, 1995: 887-944.
31. Kluft C, Jie AF, Sprengers ED, Verheijen JH. Identification of a reversible inhibitor of plasminogen activators in blood plasma. *FEBS Lett.* 1985;190:315-318.
32. Sprengers ED, Kluft C. Plasminogen activator inhibitors. *Blood.* 1987;69:381-387.
33. Dawson SJ, Wiman B, Hamsten A, Green F, Humphries S, Henney AM. The two allele sequences of a common polymorphism in the promoter of the plasminogen activator inhibitor-1 (PAI-1) gene respond differently to interleukin-1 in HepG2 cells. *J Biol Chem.* 1993;268:10739-10745.
34. Eriksson P, Kallin B, 't Hooft FM, Bavenholm P, Hamsten A. Allele-specific increase in basal transcription of the plasminogen-activator inhibitor 1 gene is associated with myocardial infarction. *Proc Natl Acad Sci U S A.* 1995;92:1851-1855.
35. Boekholdt SM, Bijsterveld NR, Moons AH, Levi M, Buller HR, Peters RJ. Genetic variation in coagulation and fibrinolytic proteins and their relation with acute myocardial infarction: a systematic review. *Circulation.* 2001;104:3063-3068.
36. Sartori MT, Wiman B, Vettore S, Dazzi F, Girolami A, Patrassi GM. 4G/5G polymorphism of PAI-1 gene promoter and fibrinolytic capacity in patients with deep vein thrombosis. *Thromb Haemost.* 1998:80:956-960.
37. Grubic N, Stegnar M, Peternel P, Kaider A, Binder BR. A novel G/A and the 4G/5G polymorphism within the promoter of the plasminogen activator inhibitor-1 gene in patients with deep vein thrombosis. *Thromb Res.* 1996;84:431-443.
38. Ridker PM, Hennekens CH, Lindpaintner K, Stampfer MJ, Miletich JP. Arterial and venous thrombosis is not associated with the 4G/5G polymorphism in the promoter of the plasminogen activator inhibitor gene in a large cohort of US men. *Circulation.* 1997;95:59-62.
39. Stegnar M, Uhrin P, Petemel P, Mavri A, Salobir-Pajnic B, Stare J, Binder BR. The 4G/5G sequence polymorphism in the promoter of plasminogen activator inhibitor-1 (PAI-1) gene: relationship to plasma PAI-1 level in venous thromboembolism. *Thromb Haemost.* 1998;79:975-979.
40. Segui R, Estelles A, Mira Y et al. PAI-1 promoter 4G/5G genotype as an additional risk factor for venous thrombosis in subjects with genetic thrombophilic defects. *Br J Haematol.* 2000; 111:122-128.
41. den Heijer M, Koster T, Blom HJ, Bos GM, Briet E, Reitsma PH, Vandenbroucke JP, Rosendaal FR. Hyperhomocysteinemia as a risk factor for deep-vein thrombosis. *N Engl J Med.* 1996;334:759-762.
42. den Heijer M, Rosendaal FR, Blom HJ, Gerrits WB, Bos GM. Hyperhomocysteinemia and venous thrombosis: a meta-analysis. *Thromb Haemost.* 1998;80:874-877.
43. den Heijer M, Brouwer IA, Bos GM et al. Vitamin supplementation reduces blood homocysteine levels: a controlled trial in patients with venous thrombosis and healthy volunteers. *Arterioscler Thromb Vasc Biol.* 1998;18:356-361.
44. Kang SS, Wong PW, Susmano A, Sora J, Norusis M, Ruggie N. Thermolabile methylenetetrahydrofolate reductase: an inherited risk factor for coronary artery disease. *Am J Hum Genet.* 1991 ;48:536-545.
45. Hanson NQ, Aras O, Yang F, Tsai MY. C677T and A1298C polymorphisms of the methylenetetrahydrofolate reductase gene: incidence and effect of combined genotypes on plasma fasting and postmethionine load homocysteine in vascular disease. *Clin Chem.* 2001:47:661-666.
46. Jacques PF, Bostom AG, Williams RR, Ellison RC, Eckfeldt JH, Rosenberg IH, Selhub J, Rozen R. Relation between folate status, a common mutation in methylenetetrahydrofolate reductase, and plasma homocysteine concentrations. *Circulation.* 1996;93:7-9.
47. Margaglione M, D'Andrea G, d'Addedda M et al. The methylenetetrahydrofolate reductase TT677 genotype is associated with venous thrombosis independently of the coexistence of the FV Leiden and the prothrombin A20210 mutation. *Thromb Haemost.* 1998;79:907-911.
48. Gemmati D, Serino ML, Trivellato C, Fiorini S, Scapoli GL. C677T substitution in the methylenetetrahydrofolate reductase gene as a risk factor for venous thrombosis and arterial disease in selected patients. *Haematologica.* 1999;84:824-828.
49. Cattaneo M, Tsai MY, Bucciarelli P, Taioli E, Zighetti ML, Bignell M, Mannucci PM. A common mutation in the methylenetetrahydrofolate reductase gene (C677T) increases the risk for deep-vein thrombosis in patients with mutant factor V (factor V:Q506). *Arterioscier Thromb Vas*c *Biol.* 1997;17:1662-1666.
50. Kluijtmans LA, den Heijer M, Reitsma PH, Heil SG, Blom HJ, Rosendaal FR. Thermolabile methylenetetrahydrofolate reductase and factor V Leiden in the risk of deep-vein thrombosis. *Thromb Haemost.* 1998;79:254-258.
51. van der Put NM, Gabreels F, Stevens EM, Smeitink JA, Trijbels FJ, Eskes TK, Van Den Heuvel LP, Blom HJ. A second common mutation in the methylenetetrahydrofolate reductase gene: an additional risk factor for neural-tube defects? *Am J Hum Genet.* 1998;62:1044-1051.
52. Weisberg 1, Tran P, Christensen B, Sibani S, Rozen R. A second genetic polymorphism in methylenetetrahydrofolate reductase (MTHFR) associated with decreased enzyme activity. *Mol Genet Metab.* 1998;64:169-172.
53. Sebastio G, Sperandeo MP, Panico M, de Franchis R, Kraus JP, Andria G. The molecular basis of homocystinuria due to cystathionine beta-synthase deficiency in Italian families, and report of four novel mutations. *Am J Hum Genet.* 1995:56:1324-1333.
54. Leclerc D, Campeau E, Goyette P et al. Human methionine synthase: cDNA cloning and identification of mutations in patients of the cblG complementation group of folate/cobalamin disorders. *Hum Mol* Genet. 1996;5: 1867-1874.
55. Wang XL, Cai H, Cranney G, Wilcken DE. The frequency of a common mutation of the methionine synthase gene in the Australian population and its relation to smoking and coronary artery disease. *J Cardiovasc Risk.* 1998;5:289-295.
56. Ma J, Stampfer MJ, Christensen B et al. A polymorphism of the methionine synthase gene: association with plasma folate, vitamin B12, homocyst(e)ine, and colorectal cancer risk. *Cancer Epidemiol Biomarkers Prev.* 1999;8:825-829.
57. Dekou V, Gudnason V, Hawe E, Miller GJ, Stansbie D, Humphries SE. Gene-environment and gene-gene interaction in the determination of plasma homocysteine levels in healthy middle-aged men. *Thromb Haemost.* 2001;85:67-74.
58. Bertina RM, Reitsma PH. A method for screening for the presence of a genetic defect associated with thrombosis and/or poor anticoagulant response to activated protein C. patent EP 0696 325 B1. 1998 May 1998.
59. Sambrook J, Rüssel DW. Molecular cloning: a laboratory manual. 3 ed. New York, USA: Cold Spring Harbor Laboratory Press, 2001.
60. Ulvik A, Ueland PM. Single nucleotide polymorphism (SNP) genotyping in unprocessed whole blood and serum by real-time PCR: application to SNPs affecting homocysteine and folate metabolism. *Clin Chem.* 2001 ;47:2050-2053.
61. Song L, O'Kane D, Krajnik KL et al. Simplified mutation detection. patent WO 99/36574. Jan 1999.
62. Fulton RJ, McDade RL, Smith PL, Kienker LJ, Kettman JR, Jr. Advanced multiplexed analysis with the FlowMetrix system. *Clin Chem.* 1997:43:1749-1756.
63. Southern EM, Mitchell AR. Chromatography of nucleic acid digests on thin layers of cellulose impregnated with polyethyleneimine. *Biochem J.* 1971;123:613-617.
64. Higuchi R, Dollinger G, Walsh PS, Griffith R. Simultaneous amplification and detection of specific DNA sequences. *Biotechnology (N Y ).* 1992;10:413417.
65. Barany F. Genetic disease detection and DNA amplification using cloned thermostable ligase. *Proc Natl Acad Sci U S A.* 1991:88:189-193.
66. Sanger F, Nicklen S, Coulson AR. DNA sequencing with chain-terminating inhibitors. *Proc Natl Acad Sci U S A.* 1977;74:5463-5467.
67. Fu DJ, Broude NE, Koster H, Smith CL, Cantor CR. Efficient preparation of short DNA sequence ladders potentially suitable for MALDI-TOF DNA sequencing. *Genet Anal.* 1996;12:137-142.
68. Brockmann JM. A multistep chemical modification procedure to create DNA arrays on gold surfaces for the study of protein-DNA interactions with surface plasmon resonance imaging. Journal of the American Chemical Society 121, 8044-8051. 1999.
69. Barie N, Rapp M, Ache HJ. UV-crosslinked polysiloxanes as new coating materials for SAW devices with long-term stability. Sensors and Actuators B 46, 97-103. 1998.
70. Gauglitz G, Brecht A, Kraus G, Nahm W. Chemical and biochemical sensors based on interferometry at thin multilayers. Sensors and Actuators B 11, 21-27. 1993.
71. Shchepinov MS, Udalova IA, Bridgman A, Southern EM. Oligonucleotide dendrimers: synthesis and use as polylabeled DNA probels. Nucleic Acids Research 25, 4447-4454. 1997.
72. Stomakhin AA, Vasiliskov VA, Timofeev E, Schulga D, Cotter RJ, Mirzabekov AD. DNA sequence analysis by hybridization with oligonucleotide microchips: MALDI mass spectrometry of 5mers contiguously stacked to microchip oligonucleotides. Nucleic Acids Research 28, 1193-1198.2000.
73. van de Riijke F, Zijlmans H, Li S, Vail T, Raap AK, Niedbala RS, Tanke HJ. Up-converting phophor reporters for nucleic acid microarrays. Biotechnology 19, 273-276. 2001.
74. Sun B, Xie W, Yi G, Chen D, Zhou Y, Cheng J. Microminiaturized immunoassays using quantum dots as fluorescent label by laser confocal scanning fluorescence detection. Journal Of Immunological Methods 249, 85-89. 2001.

SEQUENCE LISTING

<110>  Ogham GmbH

<120>  Methode zur Bestimmung eines erblichen Thromboserisikos mi
t DNA-arrays

<130>  45341

<150>  DE 10237073.7
<151>  2002-08-09

<160>  154

<170>  PatentIn version 3.1

<210>  1
<211>  24
<212>  DNA
<213>  Homo sapiens

<400>  1
agaaaatgat gcccagtgct taac
     24

<210>  2
<211>  25
<212>  DNA
<213>  Homo sapiens

<400>  2
ccattattta gccaggagac ctaac
     25

<210>  3
<211>  20
<212>  DNA
<213>  Homo sapiens

<400>  3
tgggatgcag gcttacattg
     20

<210>  4
<211>  23
<212>  DNA
<213>  Homo sapiens

<400> 4
agtggtatga accccaacaa ctc
23

<210> 5
<211> 24
<212> DNA
<213> Homo sapiens

<400> 5
acagaaggtc attgatcagt ttgg
24

<210> 6
<211> 23
<212> DNA
<213> Homo sapiens

<400> 6
accaggtggt ggattcttaa gtc
23

<210> 7
<211> 24
<212> DNA
<213> Homo sapiens

<400> 7
ctctcctgac tgtcatccct attg
24

<210> 8
<211> 20
<212> DNA
<213> Homo sapiens

<400> 8
cacctggatg ggaaagatcc
20

<210> 9
<211> 18
<212> DNA
<213> Homo sapiens

<400> 9
acgatggggc aagtgatg
18

<210> 10
<211> 19
<212> DNA
<213> Homo sapiens

<400> 10
gtgcatgcct tcacaaagc
19

<210> 11
<211> 23
<212> DNA
<213> Homo sapiens

<400> 11
tggggagctg aaggactact acc
23

<210> 12
<211> 20
<212> DNA
<213> Homo sapiens

<400> 12
caggatgggg aagtcacagc
20

<210> 13
<211> 24
<212> DNA
<213> Homo sapiens

<400> 13
aatagaatat cgaggcatgt ccag
24

<210> 14
<211> 21
<212> DNA
<213> Homo sapiens

<400>  14
actcgaccta ccgtcctgtc c
     21

<210>  15
<211>  18
<212>  DNA
<213>  Homo sapiens

<400>  15
agcaccgtgg ggatgaag
     18

<210>  16
<211>  20
<212>  DNA
<213>  Homo sapiens

<400>  16
cgtggggatg aagtcgtagc
     20

<210>  17
<211>  21
<212>  DNA
<213>  Homo sapiens

<400>  17
gttgtctgct ccgtctggtt c
     21

<210>  18
<211>  20
<212>  DNA
<213>  Homo sapiens

<400>  18
cagcacacct ccaacctcag
     20

<210>  19
<211>  24
<212>  DNA
<213>  Homo sapiens

<400> 19
ccacgtgatt gtctaggttt tgtc
24

<210> 20
<211> 27
<212> DNA
<213> Homo sapiens

<400> 20
ctacaccagt tttatcatct tttgctc
27

<210> 21
<211> 26
<212> DNA
<213> Homo sapiens

<400> 21
aacaagcaaa aatctgtttc taccac
26

<210> 22
<211> 22
<212> DNA
<213> Homo sapiens

<400> 22
tggctccttc aaactcaaca ag
22

<210> 23
<211> 19
<212> DNA
<213> Homo sapiens

<400> 23
ttcggcttcc ccgacttag
19

<210> 24
<211> 18
<212> DNA
<213> Homo sapiens

<400>  24
tgtattcctc gcctgtcc
     18

<210>  25
<211>  18
<212>  DNA
<213>  Homo sapiens

<400>  25
tgtattcctt gcctgtcc
     18

<210>  26
<211>  19
<212>  DNA
<213>  Homo sapiens

<400>  26
ctgtattcct tgcctgtcc
     19

<210>  27
<211>  18
<212>  DNA
<213>  Homo sapiens

<400>  27
tgtattccta gcctgtcc
     18

<210>  28
<211>  19
<212>  DNA
<213>  Homo sapiens

<400>  28
ctgtattcct agcctgtcc
     19

<210>  29
<211>  18
<212>  DNA
<213>  Homo sapiens

<400>  29
tgtattcctg gcctgtcc
      18

<210>  30
<211>  22
<212>  DNA
<213>  Homo sapiens

<400>  30
tcttaagatt cctggttttc tt
      22

<210>  31
<211>  22
<212>  DNA
<213>  Homo sapiens

<400>  31
tcttaagatt cgtggttttc tt
      22

<210>  32
<211>  22
<212>  DNA
<213>  Homo sapiens

<400>  32
tcttaagatt catggttttc tt
      22

<210>  33
<211>  22
<212>  DNA
<213>  Homo sapiens

<400>  33
tcttaagatt cttggttttc tt
      22

<210>  34
<211>  18
<212>  DNA
<213>  Homo sapiens

<400>  34
attgaggctc gctgagag
      18

<210>  35
<211>  18
<212>  DNA
<213>  Homo sapiens

<400>  35
attgaggctt gctgagag
      18

<210>  36
<211>  19
<212>  DNA
<213>  Homo sapiens

<400>  36
attgaggctt gctgagagt
      19

<210>  37
<211>  18
<212>  DNA
<213>  Homo sapiens

<400>  37
attgaggcta gctgagag
      18

<210>  38
<211>  19
<212>  DNA
<213>  Homo sapiens

<400>  38
attgaggcta gctgagagt
      19

<210>  39
<211>  18
<212>  DNA
<213>  Homo sapiens

<400>  39
attgaggctg gctgagag
     18

<210>  40
<211>  17
<212>  DNA
<213>  Homo sapiens

<400>  40
gctgactccc cacgtgt
     17

<210>  41
<211>  17
<212>  DNA
<213>  Homo sapiens

<400>  41
ctgactcccc acgtgtc
     17

<210>  42
<211>  16
<212>  DNA
<213>  Homo sapiens

<400>  42
ctgactcccc acgtgt
     16

<210>  43
<211>  17
<212>  DNA
<213>  Homo sapiens

<400>  43
ctgactcccc cacgtgt
     17

<210>  44
<211>  17
<212>  DNA
<213>  Homo sapiens

<400> 44
ctgactccac cacgtgt
17

<210> 45
<211> 18
<212> DNA
<213> Homo sapiens

<400> 45
gctgactcca ccacgtgt
18

<210> 46
<211> 17
<212> DNA
<213> Homo sapiens

<400> 46
ctgactcctc cacgtgt
17

<210> 47
<211> 18
<212> DNA
<213> Homo sapiens

<400> 47
gctgactcct ccacgtgt
18

<210> 48
<211> 17
<212> DNA
<213> Homo sapiens

<400> 48
ctgactccgc cacgtgt
17

<210> 49
<211> 16
<212> DNA
<213> Homo sapiens

<400> 49
ggctgactcc ccacgt
16

<210> 50
<211> 16
<212> DNA
<213> Homo sapiens

<400> 50
gctgactccc ccacgt
16

<210> 51
<211> 17
<212> DNA
<213> Homo sapiens

<400> 51
ggctgactcc accacgt
17

<210> 52
<211> 16
<212> DNA
<213> Homo sapiens

<400> 52
gctgactcca ccacgt
16

<210> 53
<211> 17
<212> DNA
<213> Homo sapiens

<400> 53
ggctgactcc tccacgt
17

<210> 54
<211> 16
<212> DNA
<213> Homo sapiens

<400> 54
gctgactcct ccacgt
16

<210> 55
<211> 16
<212> DNA
<213> Homo sapiens

<400> 55
gctgactccg ccacgt
16

<210> 56
<211> 16
<212> DNA
<213> Homo sapiens

<400> 56
atgaaatcgg ctcccg
16

<210> 57
<211> 16
<212> DNA
<213> Homo sapiens

<400> 57
aaatcggctc ccgcag
16

<210> 58
<211> 17
<212> DNA
<213> Homo sapiens

<400> 58
gatgaaatcg actcccg
17

<210> 59
<211> 16
<212> DNA
<213> Homo sapiens

<400>  59
atgaaatcga ctcccg
     16

<210>  60
<211>  17
<212>  DNA
<213>  Homo sapiens

<400>  60
gaaatcgact cccgcag
     17

<210>  61
<211>  16
<212>  DNA
<213>  Homo sapiens

<400>  61
aaatcgactc ccgcag
     16

<210>  62
<211>  17
<212>  DNA
<213>  Homo sapiens

<400>  62
gatgaaatcg tctcccg
     17

<210>  63
<211>  16
<212>  DNA
<213>  Homo sapiens

<400>  63
atgaaatcgt ctcccg
     16

<210>  64
<211>  17
<212>  DNA
<213>  Homo sapiens

<400> 64
gaaatcgtct cccgcag
17

<210> 65
<211> 16
<212> DNA
<213> Homo sapiens

<400> 65
aaatcgtctc ccgcag
16

<210> 66
<211> 16
<212> DNA
<213> Homo sapiens

<400> 66
atgaaatcgc ctcccg
16

<210> 67
<211> 16
<212> DNA
<213> Homo sapiens

<400> 67
aaatcgcctc ccgcag
16

<210> 68
<211> 22
<212> DNA
<213> Homo sapiens

<400> 68
aaagacactt tcttcactgg tc
22

<210> 69
<211> 22
<212> DNA
<213> Homo sapiens

<400> 69
caaagacact ttcttcactg gt
22

<210> 70
<211> 21
<212> DNA
<213> Homo sapiens

<400> 70
aaagacactt gcttcactgg g
21

<210> 71
<211> 22
<212> DNA
<213> Homo sapiens

<400> 71
aaagacactt gctctactgg tc
22

<210> 72
<211> 22
<212> DNA
<213> Homo sapiens

<400> 72
aaagacactt acttcactgg tc
22

<210> 73
<211> 21
<212> DNA
<213> Homo sapiens

<400> 73
aaagacactt ccttcactgg t
21

<210> 74
<211> 16
<212> DNA
<213> Homo sapiens

<400>  74
acccttcggg atccac
      16

<210>  75
<211>  15
<212>  DNA
<213>  Homo sapiens

<400>  75
ttcgggatcc acccc
      15

<210>  76
<211>  17
<212>  DNA
<213>  Homo sapiens

<400>  76
ccccaatgat ctgcaga
      17

<210>  77
<211>  16
<212>  DNA
<213>  Homo sapiens

<400>  77
aaaagcccca cctgga
      16

<210>  78
<211>  18
<212>  DNA
<213>  Homo sapiens

<400>  78
cccacctgga tgatccac
      18

<210>  79
<211>  17
<212>  DNA
<213>  Homo sapiens

<400> 79
cccacctgga tgatcca
17

<210> 80
<211> 17
<212> DNA
<213> Homo sapiens

<400> 80
gccccacctg gatgatc
17

<210> 81
<211> 18
<212> DNA
<213> Homo sapiens

<400> 81
cccaatgatc tgcagagg
18

<210> 82
<211> 18
<212> DNA
<213> Homo sapiens

<400> 82
ccccaatgat ctgcagag
18

<210> 83
<211> 18
<212> DNA
<213> Homo sapiens

<400> 83
caccccaatg atctgcag
18

<210> 84
<211> 18
<212> DNA
<213> Homo sapiens

<400> 84
cccagtgatc tgcagagg
18

<210> 85
<211> 18
<212> DNA
<213> Homo sapiens

<400> 85
ccccagtgat ctgcagag
18

<210> 86
<211> 18
<212> DNA
<213> Homo sapiens

<400> 86
caccccagtg atctgcag
18

<210> 87
<211> 24
<212> DNA
<213> Homo sapiens

<400> 87
gactcataat ggtcctgtct aata
24

<210> 88
<211> 23
<212> DNA
<213> Homo sapiens

<400> 88
gagactcata atggtcctgt cta
23

<210> 89
<211> 24
<212> DNA
<213> Homo sapiens

<400>  89
cataatggtc ctgtctaata tctt
     24

<210>  90
<211>  24
<212>  DNA
<213>  Homo sapiens

<400>  90
gactcataat ggccctgtct aata
     24

<210>  91
<211>  23
<212>  DNA
<213>  Homo sapiens

<400>  91
actcataatg gccctgtcta ata
     23

<210>  92
<211>  22
<212>  DNA
<213>  Homo sapiens

<400>  92
actcataatg gccctgtcta at
     22

<210>  93
<211>  23
<212>  DNA
<213>  Homo sapiens

<400>  93
gagactcata atggccctgt cta
     23

<210>  94
<211>  22
<212>  DNA
<213>  Homo sapiens

<400> 94
agactcataa tggccctgtc ta
22

<210> 95
<211> 22
<212> DNA
<213> Homo sapiens

<400> 95
gagactcata atggccctgt ct
22

<210> 96
<211> 24
<212> DNA
<213> Homo sapiens

<400> 96
gactcataat ggacctgtct aata
24

<210> 97
<211> 23
<212> DNA
<213> Homo sapiens

<400> 97
gactcataat ggacctgtct aat
23

<210> 98
<211> 23
<212> DNA
<213> Homo sapiens

<400> 98
actcataatg gacctgtcta ata
23

<210> 99
<211> 23
<212> DNA
<213> Homo sapiens

<400>  99
gagactcata atggacctgt cta
     23

<210>  100
<211>  22
<212>  DNA
<213>  Homo sapiens

<400>  100
agactcataa tggacctgtc ta
     22

<210>  101
<211>  22
<212>  DNA
<213>  Homo sapiens

<400>  101
gagactcata atggacctgt ct
     22

<210>  102
<211>  24
<212>  DNA
<213>  Homo sapiens

<400>  102
cataatggac ctgtctaata tctt
     24

<210>  103
<211>  23
<212>  DNA
<213>  Homo sapiens

<400>  103
ataatggacc tgtctaatat ctt
     23

<210>  104
<211>  23
<212>  DNA
<213>  Homo sapiens

<400>  104
cataatggac ctgtctaata tct
      23



<210>  105
<211>  24
<212>  DNA
<213>  Homo sapiens

<400>  105
gactcataat gggcctgtct aata
      24



<210>  106
<211>  23
<212>  DNA
<213>  Homo sapiens

<400>  106
actcataatg ggcctgtcta ata
      23



<210>  107
<211>  22
<212>  DNA
<213>  Homo sapiens

<400>  107
actcataatg ggcctgtcta at
      22



<210>  108
<211>  23
<212>  DNA
<213>  Homo sapiens

<400>  108
gagactcata atgggcctgt cta
      23



<210>  109
<211>  22
<212>  DNA
<213>  Homo sapiens

<400>  109
agactcataa tgggcctgtc ta
      22

<210>  110
<211>  21
<212>  DNA
<213>  Homo sapiens

<400>  110
agactcataa tgggcctgtc t
      21

<210>  111
<211>  24
<212>  DNA
<213>  Homo sapiens

<400>  111
cataatgggc ctgtctaata tctt
      24

<210>  112
<211>  23
<212>  DNA
<213>  Homo sapiens

<400>  112
ataatgggcc tgtctaatat ctt
      23

<210>  113
<211>  22
<212>  DNA
<213>  Homo sapiens

<400>  113
ataatgggcc tgtctaatat ct
      22

<210>  114
<211>  25
<212>  DNA
<213>  Homo sapiens

<400> 114
acttgtccat attcgctggt attac
25

<210> 115
<211> 21
<212> DNA
<213> Homo sapiens

<400> 115
gtcccaaatg acttcctctg c
21

<210> 116
<211> 25
<212> DNA
<213> Homo sapiens

<400> 116
ccagaagaaa ttctcagaat ttctg
25

<210> 117
<211> 24
<212> DNA
<213> Homo sapiens

<400> 117
tccacgtcac tgtagtatgg tctt
24

<210> 118
<211> 23
<212> DNA
<213> Homo sapiens

<400> 118
gtattactgg ctcttcctga gcc
23

<210> 119
<211> 23
<212> DNA
<213> Homo sapiens

<400>  119
aaataattct ttcacgggat tgg
     23

<210>  120
<211>  23
<212>  DNA
<213>  Homo sapiens

<400>  120
aacagaggac tcttggtctt tcc
     23

<210>  121
<211>  24
<212>  DNA
<213>  Homo sapiens

<400>  121
ctctcttgtg tcaacaacct tgtc
     24

<210>  122
<211>  21
<212>  DNA
<213>  Homo sapiens

<400>  122
ttcacaaagc ggaagaatgt g
     21

<210>  123
<211>  21
<212>  DNA
<213>  Homo sapiens

<400>  123
cttcaggtca gcctcaaagc t
     21

<210>  124
<211>  23
<212>  DNA
<213>  Homo sapiens

<400> 124
gggacttgct cttcaggtag aag
23

<210> 125
<211> 22
<212> DNA
<213> Homo sapiens

<400> 125
ccactccagc atcactcact tt
22

<210> 126
<211> 20
<212> DNA
<213> Homo sapiens

<400> 126
accgtgggga tgaagtcgta
20

<210> 127
<211> 20
<212> DNA
<213> Homo sapiens

<400> 127
ttgtctgctc cgtctggttc
20

<210> 128
<211> 18
<212> DNA
<213> Homo sapiens

<400> 128
gcggcttcag ggctcaag
18

<210> 129
<211> 27
<212> DNA
<213> Homo sapiens

<400> 129
cctcaaagta ttcatccttt agatttt
27

<210> 130
<211> 23
<212> DNA
<213> Homo sapiens

<400> 130
ctaacagctg ggaacactga aaa
23

<210> 131
<211> 29
<212> DNA
<213> Homo sapiens

<400> 131
gcatcaaaaa gactcctaag aaggtaaag
29

<210> 132
<211> 28
<212> DNA
<213> Homo sapiens

<400> 132
gcatcaaaaa gactcctaag aaggtaaa
28

<210> 133
<211> 27
<212> DNA
<213> Homo sapiens

<400> 133
gcatcaaaaa gactcctaag aaggtaa
27

<210> 134
<211> 26
<212> DNA
<213> Homo sapiens

<400>  134
gcatcaaaaa gactcctaag aaggta
     26

<210>  135
<211>  25
<212>  DNA
<213>  Homo sapiens

<400>  135
gcatcaaaaa gactcctaag aaggt
     25

<210>  136
<211>  24
<212>  DNA
<213>  Homo sapiens

<400>  136
gcatcaaaaa gactcctaag aagg
     24

<210>  137
<211>  23
<212>  DNA
<213>  Homo sapiens

<400>  137
gcatcaaaaa gactcctaag aag
     23

<210>  138
<211>  22
<212>  DNA
<213>  Homo sapiens

<400>  138
gcatcaaaaa gactcctaag aa
     22

<210>  139
<211>  21
<212>  DNA
<213>  Homo sapiens

<400> 139
gcatcaaaaa gactcctaag a
21

<210> 140
<211> 20
<212> DNA
<213> Homo sapiens

<400> 140
gcatcaaaaa gactcctaag
20

<210> 141
<211> 19
<212> DNA
<213> Homo sapiens

<400> 141
gcatcaaaaa gactcctaa
19

<210> 142
<211> 18
<212> DNA
<213> Homo sapiens

<400> 142
gcatcaaaaa gactccta
18

<210> 143
<211> 17
<212> DNA
<213> Homo sapiens

<400> 143
gcatcaaaaa gactcct
17

<210> 144
<211> 16
<212> DNA
<213> Homo sapiens

<400> 144
gcatcaaaaa gactcc
16

<210> 145
<211> 25
<212> DNA
<213> Homo sapiens

<400> 145
ggtgaaaaca cctcagccaa aaaaa
25

<210> 146
<211> 24
<212> DNA
<213> Homo sapiens

<400> 146
ggtgaaaaca cctcagccaa aaaa
24

<210> 147
<211> 23
<212> DNA
<213> Homo sapiens

<400> 147
ggtgaaaaca cctcagccaa aaa
23

<210> 148
<211> 22
<212> DNA
<213> Homo sapiens

<400> 148
ggtgaaaaca cctcagccaa aa
22

<210> 149
<211> 21
<212> DNA
<213> Homo sapiens

<400> 149
ggtgaaaaca cctcagccaa a
21

<210> 150
<211> 20
<212> DNA
<213> Homo sapiens

<400> 150
ggtgaaaaca cctcagccaa
20

<210> 151
<211> 19
<212> DNA
<213> Homo sapiens

<400> 151
ggtgaaaaca cctcagcca
19

<210> 152
<211> 18
<212> DNA
<213> Homo sapiens

<400> 152
ggtgaaaaca cctcagcc
18

<210> 153
<211> 17
<212> DNA
<213> Homo sapiens

<400> 153
ggtgaaaaca cctcagc
17

<210> 154
<211> 16
<212> DNA
<213> Homo sapiens

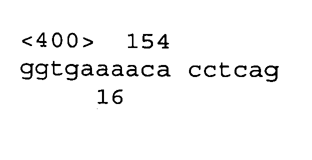

## Patentansprüche

1. Verfahren zum Bestimmen der genetischen Disposition eines Patienten zur Thromboseneigung, **gekennzeichnet durch** die Untersuchung mindestens zweier Nukleinsäureabschnitte aus dem Genom des Patienten und die anschließende multifaktorielle Auswertung der Untersuchungsergebnisse, wobei die Nukleinsäureabschnitte allelische Polymorphismus umfassen können, deren Vorhandensein im menschlichen Genom mit einer erhöhten Thromboseneigung korreliert.

2. Verfahren nach Anspruch 1, **gekennzeichnet durch** die Untersuchung mindestens zweier allelischer Polymorphismen aus der folgenden Gruppe: FakV-Leiden; FakV-Cambrdige, Fakll-G20210A, PAI1-4G/5G, MTHFR-C677T, MTHFR-A1298C, CBSins68bp, MTR-A2756G.

3. Verfahren nach Anspruch 1 oder 2, **dadurch gekennzeichnet, daß** das Verfahren mindestens einen Schritt der Amplifikation der Nukleinsäureabschnitte umfaßt.

4. Verfahren nach Anspruch 3, **gekennzeichnet durch** eine Ligase-Kettenreaktion.

5. Verfahren nach Anspruch 3, **gekennzeichnet durch** eine PCR basierte Amplifikation.

6. Verfahren nach Anspruch 4, **gekennzeichnet durch** die Verwendung mindestens einer der Oligonukleotide der Tabelle 3 als Primer.

7. Verfahren nach Anspruch 5 oder 6, **dadurch gekennzeichnet, daß** die PCR basierte Amplifikation unter folgenden Bedingungen erfolgt:
i). Denaturierung: 1 Zyklus für 1 bis 15 min bei 92-95 °C, vorzugsweise bei 95 °C für 15 min
ii). Amplifikation: 20 bis 45 Zyklen, vorzugweise 35 Zyklen
- Denaturierung 92 - 95 °C, 1 min; vorzugsweise bei 94°C für 1 min
- Annealing bei 52 - 62 °C, 0,5 - 4 min; vorzugweise bei 56°C für 2 min
- Extension: 68 - 72 °C, 1- 5 min; vorzugweise bei 68°C für 1 min
iii). Extension: 1 Zyklus 68 - 72 °C, 5 - 30 min; vorzugweise bei 68°C für 5 min.

8. Verfahren nach einem der Ansprüche 5 bis 7, **gekennzeichnet durch** folgenden Reaktionsansätze:
a.) Primer: 100 bis 800 nM; vorzugweise 400 nM
humane genomische DNA: 20 bis 250 ng; vorzugweise 100 ng dNTPS (je): 200 bis 500 µM; vorzugweise 250 µM Taq-DNA Polymerase: 2,5 bis 10 U; vorzugweise 5 U 10 X Puffer: 5 µl
oder
b.) Primer: 100 bis 1200 nM; vorzugweise 800 nM
humane genomische DNA: 20 bis 250 ng; vorzugweise 100 ng dNTPS (je): 200 bis 500 µM; vorzugweise 200 µM AccuPrime Taq-DNA Polymerase: 2,5 bis 20 U; vorzugweise 10 U 10 X Puffer: 5 µl.

9. Verfahren nach einem der vorherigen Ansprüche, **gekennzeichnet durch** eine Identifikation der Polymorphismen **durch** Hybridisierung mit Oligonukleotiden, die entweder einen Polymorphismus aus der Tabelle 1 oder die jeweilige Wildtypsequenz aufweisen oder dazu komplementär sind.

10. Verfahren nach Anspruch 9 **gekennzeichnet durch** Oligonukleotide aus der Tabelle 5 als Sonden für die Untersuchung der Polymorphismen oder Wildtypsequenzen.

11. Verfahren einem der Ansprüche 1 bis 10, **dadurch gekennzeichnet, daß** die Oligonukleotide auf einem Array oder beads gebunden sind.

12. Verfahren nach einem der Ansprüche 1 bis 8 **gekennzeichnet durch** eine Sequenzierung der amplifizierten Nukleinsäureabschnitte zum Nachweis der Polymorphismen.

13. Oligonukleotid mit einer Nukleotidsequenz gemäß einer der Seq.lD. Nr 1 bis 154.

14. Zusammensetzung mit Oligunukleotiden aus der Tabelle 3 als Primer für die Amplifikation Thrombose-relevanter Polymorphismen.

15. Verwendung der Oligonukleotide mit einer Nukleotidsequenz aus der Tabelle 3 als Primer zur Amplifikation eines Nukleinsäureabschnitts, dessen Vorhandensein im menschlichen Genom mit einer erhöhten Thromboseneigung korreliert.
